(19) Europäisches Patentamt
European Patent Office
Office européen des brevets

(11) Publication number: **0 008 277**
**B1**

(12) # EUROPEAN PATENT SPECIFICATION

(45) Date of publication of patent specification: **02.09.81**

(21) Application number: **79810066.5**

(22) Date of filing: **30.07.79**

(51) Int. Cl.³: **C 07 D 491/052,**
A 61 K 31/505
//C07D239/62, C07C69/38,
(C07D491/052, 311/00,
239/00)

(54) **Benzopyrano derivatives, pharmaceutical compositions containing them and processes for preparing the compounds and compositions.**

(30) Priority: **31.07.78 US 929598**

(43) Date of publication of application:
**20.02.80 Bulletin 80/4**

(45) Publication of the grant of the European patent:
**02.09.81 Bulletin 81/35**

(84) Designated Contracting States:
**AT BE CH DE FR GB IT NL SE**

(56) References cited:
**none**

(73) Proprietor: **SCHERICO LTD.**
**Töpferstrasse 5**
**CH-6004 Lucerne (CH)**

(72) Inventor: **Blythin, David John**
**487 Mountain Avenue**
**Caldwell, New Jersey 07006 (US)**

(74) Representative: **Antony, Fritz, Dr. et al,**
**P.O. Box 601 Winkelriedstrasse 35**
**CH-6002 Lucerne (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 008 277 B1

# Benzopyrano derivatives, pharmaceutical compositions containing them and processes for preparing the compounds and compositions.

This invention relates to novel 2,4 - (1*H*,3*H*,5*H*) - (1) - benzopyrano - (2,3 - d) - pyrimidinediones and the pharmaceutically acceptable salts thereof, to compositions containing them and to the processes for making such compounds and compositions. The compounds are useful as anti-allergy agents in the treatment of such disease states as asthma, allergic rhinitis, urticaria and ulcerative colitis.

The novel compounds of this invention are of the formula

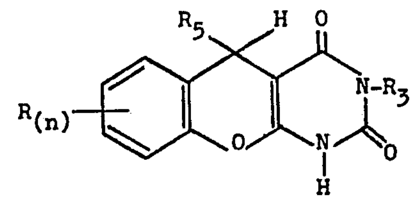

(I)

wherein each of $R_3$ and $R_5$ represents hydrogen or loweralkyl, n is an integer from one to four and R represents hydrogen, loweralkyl, lowercycloalkyl, lowercycloalkylloweralkyl, acyloxylower-alkyl, hydroxyloweralkyl, loweralkoxyloweralkyl, nitro, halogeno, haloloweralkyl, hydroxy, loweralkoxy, acylaminoloweralkyl, aminoloweralkyl, mono- or diloweralkylaminoloweralkyl, alkanoyloxy, carboxy, loweralkoxycarbonyl, acyl, formyl, cyano or a carboxamido moiety of the structure

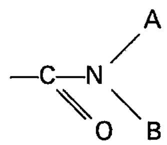

wherein A is straight or branched chain alkyl with up to 12 carbon atoms, lowercycloalkyl, lowercycloalkylloweralkyl, loweralkoxylloweralkyl, hydroxyloweralkyl, fluoroloweralkyl, loweralkenyl, loweralkylthioloweralkyl, loweralkylsulfoxylloweralkyl, loweralkylsulfonylloweralkyl, thiazolyl, oxazolyl, thiadiazolyl, methylthiadiazolyl, furyl, pyrazolyl, tetrazolyl, methyltetrazolyl, hydroxypyrimidinyl, phenyl, pyrimidinyl-dione, or the grouping

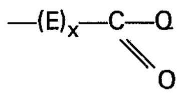

wherein E is a straight or branched chain lower alkylene or cyclic loweralkylene, X is zero or one and Q is a hydroxy, loweralkoxy, amino or mono- or diloweralkylamino; or the grouping E—$R_8$, wherein E is as defined above and is optionally substituted by hydroxy and/or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; and include the pharmaceutically acceptable salts thereof.

As employed herein, the term "halogeno" refers to fluoro, chloro, bromo and iodo. The term "lower", as it modifies radicals, such as "alkyl", "alkenyl", "alkoxy" or "cycloalkyl", defines those radicals having up to seven carbon atoms. The term "loweralkyl" includes methyl, ethyl, propyl, butyl, pentyl, hexyl and heptyl and isomers thereof, such as isopropyl, *t*-butyl, neopentyl, 2,3-dimethylbutyl and the like. "Lowercycloalkyl" includes cyclopropyl, cyclobutyl, cyclopentyl, cyclohexyl and cycloheptyl. The term "acyloxyloweralkyl" includes those radicals embraced by the partial structure

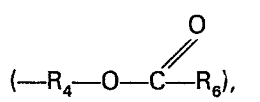

"loweralkoxyloweralkyl" includes those radicals embraced by the partial structure (—$R_4$—O—$R_1$), "haloloweralkyl" includes mono-, di- and tri-halogenated lower alkyl radicals of which —$CF_3$, —$CHF_2$, —$CH_2F$, —$CHCl_2$ and $CH_2CF_3$ are preferred, "acylaminoloweralkyl" includes those radicals embraced by the partial structure

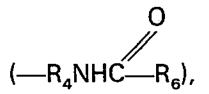

and "alkanoyloxy" includes those radicals embraced by the partial structure

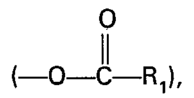

of which acetoxy is preferred, "acyl" includes those radicals embraced by the partial structure

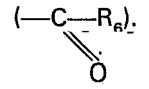

In the above partial structures $R_4$ is loweralkylene $R_6$ is a straight or branched chain alkyl radical with up to 12 carbon atoms and $R_1$ is loweralkyl.

In a preferred group of compounds of formula I n is one or two and the R substituent is located at the 7- or/and 8-position. At least one substituent in a multi-substituted compound is preferably in 7- or 8-position.

It is also preferred that both $R_3$ and $R_5$ represent hydrogen although, still within the preferred scope, one of $R_3$ and $R_5$ may represent methyl. Preferred R-substituents are halogen, carboxy, loweralkoxycarbonyl, acylaminoloweralkyl, acyloxyloweralkyl and the carboxamido moiety, as it is defined above of which loweralkoxycarbonyl, acyloxyloweralkyl and the carboxamido moiety are most preferred. Within the definition of the carboxamido moiety A is preferably straight or branched chain alkyl with up to 12 carbon atoms, hydroxyloweralkyl, tetrazolyl or the grouping —E—$R_8$, wherein E is straight chain alkylene with up to three carbon atoms and is optionally substituted by hydroxy, and $R_8$ is phenyl, tetrazolyl or pyridyl; and B is hydrogen or loweralkyl. A particularly preferred carboxamido moiety is one wherein A is tetrazolyl or the grouping —E—$R_8$ with E being methylene or ethylene and $R_8$ being phenyl or pyridyl, and B is hydrogen, said moiety being located in position 7 of the molecule.

Particularly preferred compounds are 7 - tetrazolyl - aminocarbonyl - 2,4 - (1*H*,3*H*,5*H*) - (1) - benzopyrano - (2,3 - d) - pyrimidinedione and 7 - (2 - [2 - pyridyl]ethylaminocarbonyl) - 2,4 - (1*H*,3*H*,5*H*) - (1) - benzopyrano - (2,3 - d) - pyrimidinedione.

As the compounds are acidic in character they will form physiologically acceptable (i.e., pharmaceutically acceptable) metal or amine cation salts. Illustrative examples of such metals are the alkali metal, i.e., lithium, sodium and potassium, and the alkaline earth metals, i.e., magnesium and calcium. Other metals, i.e., aluminum, zinc and iron are also within the scope of this invention. Illustrative of the amines are those derived from primary, secondary or tertiary amines. Examples of suitable amines are methylamine, dimethylamine, triethylamine, ethylamine, dibutylamine, triisopropylamine, *N*-methylhexylamine, decylamine, dodecylamine, allylamine, crotylamine, cyclopentylamine, dicyclohexylamine, benzylamine, dibenzylamine, $\alpha$-phenylethylamine, $\beta$-phenylethylamine, ethylenediamine, diethylenetriamine and like aliphatic, cycloaliphatic and araliphatic amines containing up to and including about 18 carbon atoms, as well as heterocyclic amines, i.e., piperidine, morpholine, pyrrolidine, piperazine and lower alkyl derivatives thereof, i.e., 1-methyl piperidine, 4-ethylmorpholine, 1-isopropylpyrrolidine, 2-methylpyrrolidine, 1,4-dimethylpiperazine, 2-methylpiperidine, 1,4-dimethylpiperazine, 2-methylpiperidine and the like, as well as amines containing water solubilizing or hydrophilic groups, i.e., mono, di-, and triethanolamine, ethyldiethanolamine, N-butylethanolamine, 2 - amino - 1 - butanol, 2 - amino - 2 - ethyl - 1,3 - propanediol, 2 - amino - 2 - methyl - 1 - propanol, tris (hydroxymethyl) aminomethane, *N*-phenylethanolamine, *N*-(*p*-tetramylphenyl) diethanolamine, galactamine, *N*-methylglucamine, *N*-methylglucosamine and the like.

The compounds of formula I may be prepared by applying one of the following steps A to E.

A) for the preparation of compounds of formula I, wherein $R_5$ is hydrogen; reducing a compound of the formula

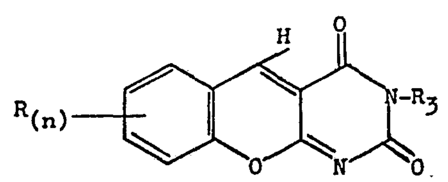

(IV)

wherein R, n and $R_3$ are as defined above; with the proviso that when R is an electron-withdrawing radical then $R_3$ is hydrogen; or

B) for the preparation of compounds of formula I, wherein $R_3$ is hydrogen and R and $R_5$ are delimited as specified in the provisos hereinbelow; cyclising a compound of the formula

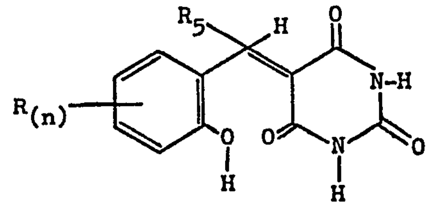

(VII)

wherein R is as defined above with the proviso that it is not acyloxyloweralkyl or alkanoyloxy; and n and $R_5$ are as defined above with the proviso that $R_5$ must be hydrogen when R is carboxy, acyl, formyl, acyl, formyl or haloloweralkyl; and wherein the hydroxy group in position 2 of the phenyl moiety may be protected; or

C) for the preparation of compounds of formula I, wherein R and n are as defined above and $R_3$ and $R_5$ are both hydrogen, reacting a compound of the formula

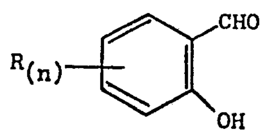

(II)

wherein R and n are as defined above, with barbituric acid in an alcoholic solvent and in the presence of a sulfonic acid; or

D) for the preparation of compounds of formula I, wherein R and n are as defined above, with the same proviso for substituent R as in process step B), and $R_3$ and $R_5$ are both hydrogen; reacting a compound of the formula

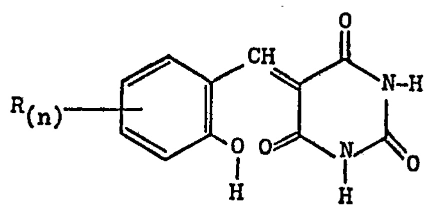

(VI)

in an alcoholic solvent and in the presence of a sulfonic acid;

E) for the preparation of compounds of formula I, wherein $R_5$ is loweralkyl; reacting a compound of the formula

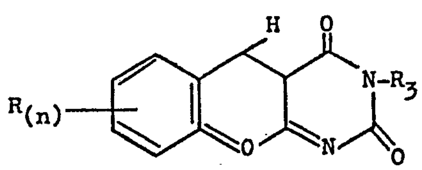

(IV)

with $R_5'$MgHal in the presence of cuprous salt, wherein R, n and $R_3$ are as defined above, $R_5$ is loweralkyl, and Hal is chlorine, bromine or iodine;

any of these steps A) to E) being followed by one or more of facultative finishing steps (i) to (xv) to convert a compound of formula I into another compound of formula I:

(i) esterification of the carboxyl group representing substituent R;
(ii) de-esterification of a loweralkoxycarbonyl group representing substituent R;
(iii) trans-esterification of a loweralkoxycarbonyl group representing substituent R;
(iv) acylation of any hydroxy group in/or representing substituent R;
(v) de-acylation of acyloxyloweralkyl, alkanoyloxy or acylaminoloweralkyl, representing substituent R;
(vi) reduction of an acyl group representing substituent R;
(vii) etherification of any hydroxyl group in/or representing substituent R;
(viii) hydrolysing, alkoholysing or reducing the cyano group representing substituent R;
(ix) oxydation of a hydroxymethyl group representing substituent R;
(x) acylation of an amino group in substituent R and, if desired, in situ or subsequently reducing the amide so obtained;
(xi) replacement of hydroxy by halogen in hydroxyloweralkyl representing substituent R;
(xii) amidation of the carboxyl group representing substituent R to obtain the carboxamido moiety as defined above;
(xiii) trans-amidation of a carboxamido moiety representing substituent R;
(xiv) de-amidation of a carboxamido moiety representing substituent R;
(xv) preparing a pharmaceutically acceptable salt of any of the compounds so obtained.

In step A) reduction of a compound of formula IV is effected by standard reduction techniques, preferably by using excess molar quantities of sodium borohydride in an alcoholic medium. The reduction is preferably effected at room temperature over a period of several days or at least until a yellow-orange coloration is discharged. Excess sodium borohydride is carefully decomposed by the addition of water and acid. If substituent R is susceptible to chemical modifications in the presence of sodium borohydride (e.g. formyl, acyl) then neutral conditions such as provided for by sodium cyanoborohydride are advantageously employed.

Alternatively, the desired compound of formula I can also be obtained from a compound of formula IV by heating the latter in the presence of a sulfonic acid, such as p-toluenesulfonic acid or methanesulfonic acid, in an alcoholic solvent, such as propanol or butanol. This process is inventive as the use of a sulfonic acid/alcohol as reducing system has not been described heretofore.

The following reaction scheme illustrates the reaction thereby showing the reaction from starting materials well known in the art:

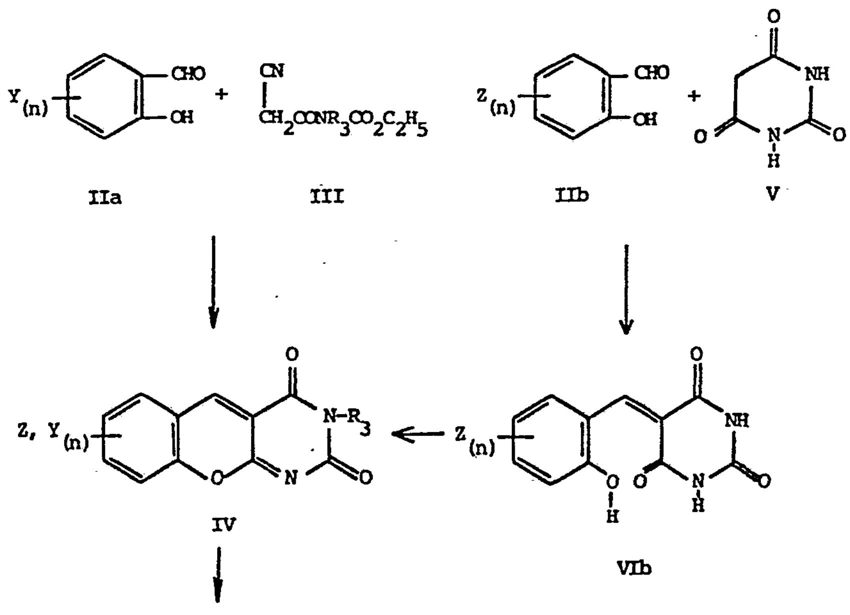

IIa III IIb V

IV VIb

I

wherein n and $R_3$ are as previously defined, Y is an electron-donating radical within the scope of R and Z is an electron-withdrawing radical within the scope of R with R being as previously defined. Electron-donating groups within the scope of R are such groups as hydrogen, loweralkyl, lowercycloalkyl, lowercycloalkylloweralkyl, acyloxyloweralkyl, hydroxyloweralky, monohalogeno, loweralkoxy, acylaminoloweralkyl, aminoloweralkyl, mono- and diloweralkylaminoloweralkyl, alkanoyloxy and loweralkoxylloweralkyl. Electron-withdrawing groups within the scope of R are such groups as loweralkoxycarbonyl, dihalogeno, nitro, carboxy, carboxamido, formyl, acyl, cyano, trifluoromethyl, difluoromethyl and the like.

In those instances wherein the initial reaction is to condense the substituted salicylaldehyde (IIa) with the cyanoacetylurethane there is produced an appropriately Y - substituted 2,4 - (3*H*) - (1) - benzopyrano - (2,3 - d) - pyrimidinedione intermediate (IV) which is isolated and may then be converted to a final product (I). The condensation is preferably effected in an alcoholic solvent, preferably ethanol, and catalytic quantities of a secondary base, preferably morpholine although other equivalently functioning bases may be employed. Generally, equimolar quantities of the reactants may be employed although in practice it is preferred to have a slight excess of the cyanoacetyl urethane. The reaction is preferably effected at reflux temperature and is continued for times up to eight hours after a yellow-orange precipitate starts to form.

In those circumstances wherein the initial reaction involves the condensation of an appropriately substituted salicylaldehyde (IIb) with barbituric acid (V), there is produced a 5 - [(2 - hydroxy) - $Z_{(n)}$ - phenylmethylene] - 2,4,6 - (1*H*,3*H*,) - pyrimidinetrione (VIb) intermediate. This condensation reaction is effected by heating an admixture of reactants at elevated temperatures (60—100°C), preferably 90°C, in an inert organic solvent (preferably aqueous dioxan) for about 0.5—12 hours. The intermediate (VIb) is then converted into intermediate (IV) by standard dehydration techniques such as employing an acid anhydride, e.g. acetic anhydride, at reflux temperature.

In the above reaction scheme, specifically in compound (IV), the phenyl substituent $R_{(N)}$ is shown as Z, $Y_{(n)}$. As per the definitions of R, Z and Y it is apparent that R and (Z,Y) are synonymous. The proviso in process step A) is also apparent from the above reaction scheme, as $R_3$ must be hydrogen if (IV) is obtained from (IIb) and (V) via (VIb).

In process step B) cyclisation of a compound (VII) is effected by standard dehydration techniques

such as by heating the compound (VII) in the presence of polyphosphoric acid or phosphorous oxychloride, or by heating the compound (VII) with sulfonic acids (preferably methanesulfonic acid) in the presence of such catalysts as phosphorous pentoxide or the like. Alternatively, a mixture of a compound (VII) and a pyridine hydrohalide is heated to melting.

The following reaction scheme illustrates the reaction thereby showing the reaction from starting materials well known in the art or described hereinabove:

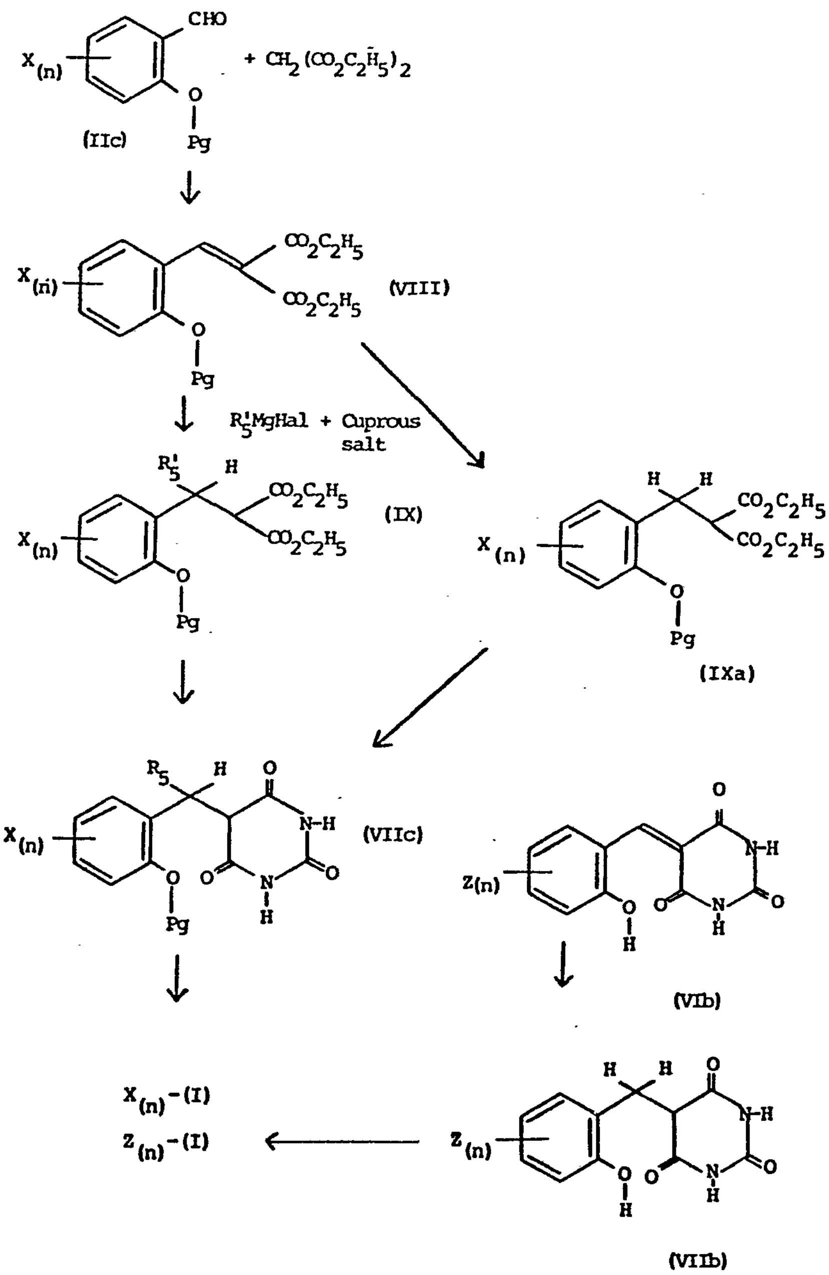

(IIc) (VIII) (IX) (IXa) (VIIc) (VIb) (VIIb)
$X_{(n)}$-(I)
$Z_{(n)}$-(I)

wherein Z, n and $R_5$ are as previously defined, $R_5$ is loweralkyl, Hal is chlorine, bromine or iodine, Pg is a hydroprotective group, such as benzyl, and X is the same as R being previously defined with the proviso that it is not acyloxyloweralkyl, haloloweralkyl, alkanoyloxy, carboxy, acyl or formyl.

In the above reaction scheme the substituted salicylaldehyde IIc, the 2-hydroxy group of which is protected, is condensed with diethyl malonate according to methods known in the art to yield the benzylidene malonate (VIII). Depending on whether a final compound (I) is desired wherein $R_5$ is hydrogen or lower alkyl ($R_5'$), the compound of formula (VIII) is either first reduced to compound (IXa) which then is reacted with urea according to a method known in the art (J.A. Vida, *et al*, *J. Med. Chem.*, 17, 732 (1974) to yield the pyrimidinetrione (VIIc) ($R_5$=H), or the compound of formula (VIII) is reacted with the Grignard reagent $R_5$MgHal in the presence of a cuprous salt, such as cuprous iodide, to introduce the desired $R_5'$ group and to yield compound (IX) which is then reacted with urea as described above for compound (IXa).

Alternatively, a compound of formula (VIb) as described in a previous reaction scheme (see page 12) is reduced to a pyrimidinetrione (VIIb). This reduction as well as the reduction of compound (VIII) is effected according to such known methods as using sodiumborohydride or, if substituent Z is susceptible to chemical modification, sodiumcyanoborohydride providing neutral conditions.

It is readily apparent from the above reaction scheme and the definitions of R, X and Z that R, if it is not acyloxyloweralkyl or alkanoyloxy, corresponds to the scope of X and Z when taken together. Thus, compounds (VIIb) and (VIIc), if taken together, are also identified herein as compounds (VII) having a R-substituent (with a proviso) rather than an X- or Z-substituent. The proviso relating to $R_5$ in process step B) is also apparent from the definition of substituent X and the above reaction scheme, as $R_5$ can only be loweralkyl in that case when the route from (IIc) via (IX) is followed.

The reaction in process step C) is inventive as such as heretofore it was completely unexpected that a substituted salicylaldehyde (II), upon reaction with barbituric acid in an alcoholic solvent, such as n-propanol, n-butanol or iso-propanol, and in the presence of a sulfonic acid, such as methanesulfonic acid or p-toluenesulfonic acid, yields compounds of formula (I) in good yields. Apparently, intermediates are formed in situ during this reaction which have the structure of compounds (VIb) and also the structure of compounds (IV). If these compounds are prepared as described in connection with the two reaction schemes above and are subjected to the reaction conditions of process step C), compounds of formula (I) are also obtained. Accordingly, in process step D) the same reaction conditions ought to be applied as described in relation to process step C) and, as it is true for process step C), also process step D) is inventive as such. The compounds of formula (VI) may be obtained in a manner shown in the above reaction scheme for compounds (VIb).

Process step E) represents a method for obtaining compounds of formula (I), wherein $R_5$ is loweralkyl, and thus, for example, is a convenient route for preparing compounds wherein both $R_3$ and $R_5$ are loweralkyl. The reaction represents the addition of a Grignard reagent, such as $CH_3MgI$, to a double bond in the presence of a cuprous salt, such as cuprous iodide. The preparation of compounds of formula (IV) is described in detail in connection with the above reaction schemes.

All of the facultative finishing steps represent chemical reactions well known in the art such as esterification, amidation, acylation and the like. They are useful when particular R-substituted compounds must be prepared and the correspondingly substituted starting materials are not available or the use thereof would reduce the overall yield.

The following examples illustrate the invention.

## Example I

### 7-Hydroxymethyl-2,4-(1*H*,3*H*,5*H*)-(1)-Benzopyrano-(2,3-d)-Pyrimidinedione

A(1) 7-Hydroxymethyl-2,4-(3*H*)-(1)-Benzopyrano-(2,3-d)-Pyrimidinedione:

To a warmed and stirred solution containing 5-hydroxy methylsalicylaldehyde (46 g) and *N*-cyanoacetylurethane (48 g) in absolute ethanol, add morpholine (0.5 ml) and reflux the resulting solution for 4 hours. After cooling, filter, ethanol wash and dry the product to yield 7 - hydroxymethyl - 2,4 - (3*H*) - (1) - benzopyrano - (2,3-d) - pyrimidinedione.

In a similar manner, by replacing the 5-hydroxymethyl salicylaldehyde with equivalent quantities of the appropriately substituted salicyaldehyde, and by substantially following the procedure of this example, there are produced the following compounds:

2,4 - (3*H*) - (1) - benzopyrano - (2,3 - d) - pyrimidinedione,
7 - methyl - 2,4 - (3*H*) - (1) - benzopyrano - (2,3 - d) - pyrimidinedione,
7 - cyclopropyl - 2,4 - (3*H*) - (1) - benzopyrano - (2,3 - d) - pyrimidinedione,
7 - *t* - butyl - 2,4 - (3*H*) - (1) - benzopyrano - (2,3 - d) - pyrimidinedione,
7,8 - dimethyl - 2,4 - (3*H*) - (1) - benzopyrano - (2,3 - d) - pyrimidinedione,
7,9 - dihydroxymethyl - 2,4 - (3*H*) - (1) - benzopyrano - (2,3 - d) - pyrimidinedione,
7 - methyl - 8 - chloro - 2,4 - (3*H*) - (1) - benzopyrano - (2,3 - d) - pyrimidinedione,
7,8 - dihydroxy - 2,4 - (3*H*) - (1) - benzopyrano - (2,3 - d) - pyrimidinedione,
6 - chloro - 2,4 - (3*H*) - (1) - benzopyrano - (2,3 - d) - pyrimidinedione,
7 - bromo - 2,4 - (3*H*) - (1) - benzopyrano - (2,3 - d) - pyrimidinedione,
9 - ethoxy - 2,4 - (3*H*) - (1) - benzopyrano - (2,3 - d) - pyrimidinedione,
7 - chloro - 2,4 - (3*H*) - (1) - benzopyrano - (2,3 - d) - pyrimidinedione,
7,8 - dichloro - 2,4 - (3*H*) - (1) - benzopyrano - (2,3 - d) - pyrimidinedione,
8 - hydroxy - 7 - aminomethyl - 2,4 - (3*H*) - (1) - benzopyrano - (2,3 - d) - pyrimidinedione,
7 - dimethylaminomethyl - 2,4 - (3*H*) - (1) - benzopyrano - (2,3 - d) - pyrimidinedione,
7 - hydroxy - 2,4 - (3*H*) - (1) - benzopyrano - (2,3 - d) - pyrimidinedione,
8 - methoxy - 2,4 - (3*H*) - (1) - benzopyrano - (2,3 - d) - pyrimidinedione.

Similarly, by replacing the *N*-cyanoacetyl urethane reactant with equivalent quantities of *N* - cyanoacetyl - *N* - methyl - urethane or *N* - cyanoacetyl - *N* - ethyl - urethane and by substantially following the foregoing reaction conditions there are produced the 3-methyl analogs or 3-ethyl analogs of the foregoing enumerated compounds.

(2) 8-chloro-2,4(3*H*)-(1)-benzopyrano-(2,3-d)-pyrimidinedione

Dissolve a mixture of 4-chlorosalicylaldehyde (6 g) and *N*-cyanoacetylurethane (6 g) in a minimum quantity of ethanol at room temperature. Add morpholine (60 mg) and allow the resulting solution to stand at room temperature overnight. Filter and wash the solid with ethanol. This product, after drying, is heated, with stirring, at 165°C for 2½ hours. Add ethanol to the solid, filter, wash with ethanol and then with ether. Dry the product to yield 8 - chloro - 2,4 - (3*H*) - (1) - benzopyrano - (2,3 - d) - pyrimidinedione.

B: 7-Hydroxymethyl-2,4-(3*H*)-(1)-benzopyrano-(2,3-d)-Pyrimidinedione

To a suspension of 7 - hydroxymethyl - 2,4 - (3*H*) - (1) - benzopyrano - (2,3 - d) - pyrimidinedione (18.8 g) in isopropanol (600 ml) add, in portions during one hour, sodium borohydride (12 g). Stir the mixture for two days at room temperature. Carefully add water to decompose the excess borohydride and then add 5.5N hydrochloric acid until the mixture is acidic. Filter, wash, consecutively with water, isopropanol, and ether, and dry the product to yield 7 - hydroxymethyl - 2,4 - (1*H*,3*H*,5*H*) - (1) - benzopyrano - (2,3 - d) - pyrimidinedione, dec. 308—309,5°C.

In a similar manner, by replacing the (3*H*) - (1) - benzopyrano - (2,3 - d) - pyrimidinedione, reactants of this part with equivalent quantities of those compounds prepared by the technique of part A of this example, and by substantially following the foregoing teachings, there are produced:

2,4 - (1*H*,3*H*,5*H*) - (1) - benzopyrano - (2,3 - d) - pyrimidinedione, dec. 310°C,
7 - methyl - 2,4 - (1*H*,3*H*,5*H*) - (1) - benzopyrano - (2,3 - d) - pyrimidinedione,
7 - cyclopropyl - 2,4 - (1*H*,3*H*,5*H*) - (1) - benzopyrano - (2,3 - d) - pyrimidinedione,
7 - *t* - butyl - 2,4 - (1*H*,3*H*,5*H*) - (1) - benzopyrano - (2,3 - d) - pyrimidinedione,
7,8 - dimethyl - 2,4 - (1*H*,3*H*,5*H*) - (1) - benzopyrano - (2,3 - d) - pyrimidinedione, m.p. > 360°C,
7,9 - dihydroxymethyl - 2,4 - (1*H*,3*H*,5*H*) - (1) - benzopyrano - (2,3 - d) - pyrimidinedione,
7 - methyl - 8 - chloro - 2,4 - (1*H*,3*H*,5*H*) - (1) - benzopyrano - (2,3 - d) - pyrimidinedione,
8 - hydroxy - 2,4 - (1*H*,3*H*,5*H*) - (1) - benzopyrano - (2,3 - d) - pyrimidinedione, chars 330°C,
7,8 - dihydroxy - 2,4 - (1*H*,3*H*,5*H*) - (1) - benzopyrano - (2,3 - d) - pyrimidinedione,
6 - chloro - 2,4 - (1*H*,3*H*,5*H*) - (1) - benzopyrano - (2,3 - d) - pyrimidinedione,
7 - bromo - 2,4 - (1*H*,3*H*,5*H*) - (1) - benzopyrano - (2,3 - d) - pyrimidinedione,
9 - ethoxy - 2,4 - (1*H*,3*H*,5*H*) - (1) - benzopyrano - (2,3 - d) - pyrimidinedione,
7 - chloro - 2,4 - (1*H*,3*H*,5*H*) - (1) - benzopyrano - (2,3 - d) - pyrimidinedione,
7 - bromo - 8 - chloro - 2,4 - (1*H*,3*H*,5*H*) - (1) - benzopyrano - (2,3 - d) - pyrimidinedione,
7,8 - dichloro - 2,4 - (1*H*,3*H*,5*H*) - (1) - benzopyrano - (2,3 - d) - pyrimidinedione,
8 - hydroxy - 7 - aminomethyl - 2,4 - (1*H*,3*H*,5*H*) - (1) - benzopyrano - (2,3 - d) - pyrimidinedione,
7 - acetoxymethyl - 2,4 - (1*H*,3*H*,5*H*) - (1) - benzopyrano - (2,3 - d) - pyrimidinedione, chars 320°C,
7 - dodecanoyloxymethyl - 2,4 - (1*H*,3*H*,5*H*) - (1) - benzopyrano - (2,3 - d) - pyrimidinedione, m.p. 229—232°C,
7 - dimethylaminomethyl - 2,4 - (1*H*,3*H*,5*H*) - (1) - benzopyrano - (2,3 - d) - pyrimidinedione,
8 - acetoxy - 2,4 - (1*H*,3*H*,5*H*) - (1) - benzopyrano - (2,3 - d) - pyrimidinedione, chars 295°C,
7 - hydroxy - 2,4 - (1*H*,3*H*,5*H*) - (1) - benzopyrano - (2,3 - d) - pyrimidinedione,
7 - (isobutyrylaminomethyl) - 8 - methoxy - 2,4 - (1*H*,3*H*,5*H*) - (1) - benzopyrano - (2,3 - d) - pyrimidinedione,
7 - (isopropylaminomethyl) - 2,4 - (1*H*,3*H*,5*H*) - (1) - benzopyrano - (2,3 - d) - pyrimidinedione,
8 - methoxy - 2,4 - (1*H*,3*H*,5*H*) - (1) - benzopyrano - (2,3 - d) - pyrimidinedione, dec. 305°C,
6,8 - dimethoxy - 2,4 - (1*H*,3*H*,5*H*) - (1) - benzopyrano - (2,3 - d) - pyrimidinedione,
and the 3-methyl and 3-ethyl homologs of the foregoing, such as 3 - ethyl - 7 - hydroxymethyl - 2,4 - (1*H*,3*H*,5*H*) - (1) - benzopyrano - (2,3 - d) - pyrimidinedione, m.p. 285°C.

## Example II
## 7-Methoxycarbonyl-2,4-(1*H*,3*H*,5*H*)-(1)-Benzopyrano-(2,3-d)-Pyrimidinedione

(A) 5 - [2′ - hydroxy - 5′ - Methoxycarbonyl - Phenyl - Methylene] - 2,4,6 - (1*H*,3*H*) - Pyrimidinetrione

On a steam bath, heat a solution of methyl 3 - formyl - 4 - hydroxybenzoate (6 g) and barbituric acid (5 g) in a minimum volume of aqueous dioxan for one hour and allow the mixture to stand at room temperature overnight. Add water, evaporate off the dioxan and filter the precipitated product. Wash the product with water and isopropanol and dry *in vacuo*.

(B) 5 - (2′ - Hydroxy - 5′ - Methoxycarbonyl) - Benzyl - 2,4,6 - (1*H*,3*H*,5*H*) - Pyrimidinedione

Suspend 5 - [(2′ - hydroxy - 5′ - methoxycarbonyl) - phenylmethylene] - 2,4,6 - (1*H*,3*H*) - pyrimidinetrione (2,3 g) in isopropanol (100 ml) with stirring and in small portions (0.2 g) add sodium borohydride (at intervals of ½ hour) until the color of the solid does not change further. After a further one hour, cautiously add water and then acidify the mixture with 5.5N hydrochloric acid. Add more

water (150 ml), evaporate off the isopropanol, and filter, water-wash and dry the resulting product.

(C) 7-Methoxycarbonyl-2,4-(1*H*,3*H*,5*H*)-(1)-Benzopyrano-(2,3-d)-Pyrimidinedione

To a stirred solution of phosphorous pentoxide (70 g) in methanesulfonic acid (600 g) at 80°C add 5 - (2′ - hydroxy - 5′ - methoxycarbonyl) - benzyl - 2,4,6 - (1*H*,3*H*,5*H*) - pyrimidinetrione (25.0 g) and keep the resulting solution at 80°C for four hours. Cool and pour the solution over ice and water. Filter and wash the precipitate with water and then with ethanol. Dry the product to yield 7 - methoxycarbonyl - 2,4 - (1*H*,3*H*,5*H*) - (1) - benzopyrano - (2,3 - d) - pyrimidinedione, dec. 320°C.

Similarly, by substituting the 3 - formyl -4 - hydroxybenzoate reactant of this example with equivalent quantities of the appropriate $R_{(n)}$ - substituted - 4 - hydroxybenzoates and by substantially following the procedure of Parts A, B and C of this example, there are produced the following compounds:

7 - carboethoxy - 2,4 - (1*H*,3*H*,5*H*) - (1) - benzopyrano - (2,3 - d) - pyrimidinedione,
6 - carbomethoxy - 2,4 - (1*H*,3*H*,5*H*) - (1) - benzopyrano - (2,3 - d) - pyrimidinedione,
8 - carbomethoxy - 2,4 - (1*H*,3*H*,5*H*) - (1) - benzopyrano - (2,3 - d) - pyrimidinedione,
9 - carbomethoxy - 2,4 - (1*H*,3*H*,5*H*) - (1) - benzopyrano - (2,3 - d) - pyrimidinedione,
7,8 - dicarbomethoxy - 2,4 - (1*H*,3*H*,5*H*) - (1) - benzopyrano - (2,3 - d) - pyrimidinedione,
7 - acetyl - 2,4 - (1*H*,3*H*,5*H*) - (1) - benzopyrano - (2,3 - d) - pyrimidinedione,
7,8 - diacetyl - 2,4 - (1*H*,3*H*,5*H*) - (1) - benzopyrano - (2,3 - d) - pyrimidinedione,
7 - butyryl - 2,4 - (1*H*,3*H*,5*H*) - (1) - benzopyrano - (2,3 - d) - pyrimidinedione,
7 - cyclobutylcarbonyl - 2,4 - (1*H*,3*H*,5*H*) - (1) - benzopyrano - (2,3 - d) - pyrimidinedione,
7,8 - dichloro - 2,4 - (1*H*,3*H*,5*H*) - (1) - benzopyrano - (2,3 - d) - pyrimidinedione,
7 - chloro - 2,4 - (1*H*,3*H*,5*H*) - (1) - benzopyrano - (2,3 - d) - pyrimidinedione,
8 - bromo - 2,4 - (1*H*,3*H*,5*H*) - (1) - benzopyrano - (2,3 - d) - pyrimidinedione,
7 - trifluoromethyl - 2,4 - (1*H*,3*H*,5*H*) - (1) - benzopyrano - (2,3 - d) - pyrimidinedione,
7 - trifluoromethyl - 8 - chloro - 2,4 - (1*H*,3*H*,5*H*) - (1) - benzopyrano - (2,3 - d) - pyrimidinedione,
7 - cyano - 2,4 - (1*H*,3*H*,5*H*) - (1) - benzopyrano - (2,3 - d) - pyrimidinedione,
7 - cyano - 8 - chloro - 2,4 - (1*H*,3*H*,5*H*) - (1) - benzopyrano - (2,3 - d) - pyrimidinedione.

## Example III
## 7-Nitro-2,4-(1*H*,3*H*,5*H*)-(1)-Benzopyrano-(2,3-d)-Pyrimidinedione

Reflux a mixture containing 5 - [(2′ - hydroxy - 5′ - nitro) - phenylmethylene - 2,4,6 - (1*H*,3*H*) - pyrimidinetrione (56 g) and acetic anhydride (1000 ml) for four hours. Filter off and wash the solid product with ether to yield 7 - nitro - 2,4 - (3*H*) - (1) - benzopyrano - (2,3 - d) - pyrimidinedione which, by following the procedure of Example I, Part B, yields the desired 7 - nitro 2,4 - (1*H*,3*H*,5*H*) - (1) - benzopyrano - (2,3 - d) - pyrimidinedione.

## Example IV
## 7-Bromo-5-Methyl-2,4-(1*H*,3*H*,5*H*)-(1)-Benzopyrano-(2,3-d)-Pyrimidinedione

(A) α-(2-Benzyloxy-5-Bromo) Phenethyl Malonate

To a cooled (—30°C) mixture of diethyl - 2 - benzyloxy - 5 - bromobenzylidene malonate (30 g) in ether (300 ml) add cuprous iodide (3.8 g) followed by a freshly prepared methyl magnesium iodide solution (from 5.1 g of magnesium and 31 g of methyl iodide in about 250 ml of ether). After the addition allow the reaction mixture to stand at room temperature for 4 hours, add a cold aqueous ammonium chloride solution and acidify with 5N HCl. Separate the organic layer and extract the aqueous layer with ethyl acetate which is combined with the separated organic layer. Dry the combined material (over $Na_2SO_4$), evaporate off the solvents to produce an oil which solidifies afterr a time.

(B) 5-[α-(2-Benzyloxy-5-Bromo)phenyl] Ethyl Barbituric Acid

α - (2 - benzyloxy - 5 - bromo)phenethyl malonate (22.5 g) is treated with urea and sodium in ethanol according to the published procedure of J.A. Vida, *et al*, *j. Med. Chem.*, *17*, 732 (1974) to yield 5 - α - (2 - benzyloxy - 5 - bromo)phenyl] ethyl barbituric acid.

(C) 7 - Bromo - 5 Methyl - 2,4 - (1*H*,3*H*,5*H*) - (1) - Benzopyrano - (2,3 - d) - Pyrimidinedione

In an atmosphere of nitrogen and with constant stirring, heat to melting a mixture of 5 - [α - (2 - benzyloxy - 5 - bromo) phenyl]ethyl barbituric acid (5 g) and pyridine hydrochloride (15 g). Heat the resulting solution at 150°C for 6 hours and after cooling, add water, filter and wash the resulting white solid with water, ethanol and then ether. Dry the product to yield 7 - bromo - 5 - methyl - 2,4 - (1*H*,3*H*,5*H*) - (1) - benzopyrano - (2,3 - d) - pyrimidinedione. Also produced by this method are:

5-methyl-2,4-(1*H*,3*H*,5*H*)-(1)-benzopyrano-(2,3-d)-pyrimidinedione, dec. 262°C,
5,7-dimethyl-2,4-(1*H*,3*H*,5*H*)-(1)-benzopyrano-(2,3-d)-pyrimidinedione,

5-methyl-7-t-butyl-2,4-(1*H*,3*H*,5*H*)-(1)-benzopyrano-(2,3-d)-pyrimidinedione,
5,7,8-trimethyl-2,4-(1*H*,3*H*,5*H*)-(1)-benzopyrano-(2,3-d)-pyrimidinedione,
5,7-dimethyl-8-chloro-2,4-(1*H*,3*H*,5*H*)-(1)-benzopyrano-(2,3-d)-pyrimidinedione,
5-methyl-6-chloro-2,4-(1*H*,3*H*,5*H*)-(1)-benzopyrano-(2,3-d)-pyrimidinedione,
5-methyl-9-ethoxy-2,4-(1*H*,3*H*,5*H*)-(1)-benzopyrano-(2,3-d)-pyrimidinedione,
5-methyl-7-chloro-2,4-(1*H*,3*H*,5*H*)-(1)-benzopyrano-(2,3-d)-pyrimidinedione,
5-methyl-7,8-dichloro-2,4-(1*H*,3*H*,5*H*)-(1)-benzopyrano-(2,3-d)-pyrimidinedione.

Example V

7-Cyano-2,4-(1*H*,3*H*,5*H*)-(1)-Benzopyrano-(2,3-d)-Pyrimidinedione

To n-butanol (40 ml) add 5-cyanosalicylaldehyde (3 g) barbituric acid (2.8 g) and methanesulfonic acid (4 ml). Stir and heat to reflux for two hours, cool, filter and wash the solid with isopropanol and ether to yield 7 - cyano - 2,4 - (1*H*,3*H*,5*H*) - (1) - benzopyrano - (2,3 - d) - pyrimidinedione.

Also, by the technique of this example there are produced:
7-nitro-2,4-(1*H*,3*H*,5*H*)-(1)-benzopyrano-(2,3-d)-pyrimidinedione,
9-methoxy-2,4-(1*H*,3*H*,5*H*)-(1)-benzopyrano-(2,3-d)-pyrimidinedione,
7-methyl-2,4-(1*H*,3*H*,5*H*)-(1)-benzopyrano-(2,3-d)-pyrimidinedione,
7-*n*-butyl-2,4-(1*H*,3*H*,5*H*)-(1)-benzopyrano-(2,3-d)-pyrimidinedione,
7-t-butyl-2,4-(1*H*,3*H*,5*H*)-(1)-benzopyrano-(2,3-d)-pyrimidinedione,
7,8-dimethyl-2,4-(1*H*,3*H*,5*H*)-(1)-benzopyrano-(2,3-d)-pyrimidinedione,
7-methyl-8-chloro-2,4-(1*H*,3*H*,5*H*)-(1)-benzopyrano-(2,3-d)-pyrimidinedione,
6-chloro-2,4-(1*H*,3*H*,5*H*)-(1)-benzopyrano-(2,3-d)-pyrimidinedione,
7-bromo-2,4-(1*H*,3*H*,5*H*)-(1)-benzopyrano-(2,3-d)-pyrimidinedione,
9-ethoxy-2,4-(1*H*,3*H*,5*H*)-(1)-benzopyrano-(2,3-d)-pyrimidinedione,
7-chloro-2,4-(1*H*,3*H*,5*H*)-(1)-benzopyrano-(2,3-d)-pyrimidinedione,
7,8-dichloro-2,4-(1*H*,3*H*,5*H*)-(1)-benzopyrano-(2,3-d)-pyrimidinedione.

Example VI

7-Methoxycarbonyl-2,4-(1*H*,3*H*,5*H*)-(1)-Benzopyrano-(2,3-d)-Pyrimidinedione

Dissolve p-toluene sulfonic acid (2 g) in sec-butanol (50 ml). Add 5 - (2' - hydroxy - 5' - methoxycarbonyl) - benzylidene - 2,4,6 - (1*H*,3*H*) - pyrimidinetrione (1.45 g), and reflux for about 18 hours. Allow to cool, add to water, filter, wash with water, isopropanol and ether consecutively to yield 7 - methoxycarbonyl - 2,4 - (1*H*,3*H*,5*H*) - (1) - benzopyrano - (2,3 - d) - pyrimidinedione, dec. 320°C.

Also, by the techniques of this example, there are produced:
7-ethoxycarbonyl-2,4-(1*H*,3*H*,5*H*)-(1)-benzopyrano-(2,3-d)-pyrimidinedione,
7-isopropoxycarbonyl-2,4-(1*H*,3*H*,5*H*)-(1)-benzopyrano-(2,3-d)-pyrimidinedione,
7-cyano-2,4-(1*H*,3*H*,5*H*)-(1)-benzopyrano-(2,3-d)-pyrimidinedione,
7-nitro-2,4-(1*H*,3*H*,5*H*)-(1)-benzopyrano-(2,3-d)-pyrimidinedione.

Example VII

9-Methoxy-2,4-(1*H*,3*H*,5*H*)-(1)-Benzopyrano-(2,3-d)-Pyrimidinedione

To a suspension of 9 - methoxy - 2,4 - (3*H*) - (1) - benzopyrano - (2,3 - d) - pyrimidinedione (24.4 g) in isopropanol (100 ml) add p-toluene sulfonic acid (6 g). Reflux for 40 hours. Cool and filter. Wash with water then with isopropanol and, finally, ether to yield 9 - methoxy - 2,4 - (1*H*,3*H*,5*H*) - (1) - benzopyrano - (2,3 - d) - pyrimidinedione.

Also, by the technique of this example, there are produced the following compounds:
7-cyclopropyl-2,4-(1*H*,3*H*,5*H*)-(1)-benzopyrano-(2,3-d)-pyrimidinedione,
7-t-butyl-2,4-(1*H*,3*H*,5*H*)-(1)-benzopyrano-(2,3-d)-pyrimidinedione,
7,8-dimethyl-2,4-(1*H*,3*H*,5*H*)-(1)-benzopyrano-(2,3-d)-pyrimidinedione,
7-methyl-8-chloro-2,4-(1*H*,3*H*,5*H*)-(1)-benzopyrano-(2,3-d)-pyrimidinedione,
6-chloro-2,4-(1*H*,3*H*,5*H*)-(1)-benzopyrano-(2,3-d)-pyrimidinedione,
7-bromo-2,4-(1*H*,3*H*,5*H*)-(1)-benzopyrano-(2,3-d)-pyrimidinedione,
9-ethoxy-2,4-(1*H*,3*H*,5*H*)-(1)-benzopyrano-(2,3-d)-pyrimidinedione,
7-chloro-2,4-(1*H*,3*H*,5*H*)-(1)-benzopyrano-(2,3-d)-pyrimidinedione,
7,8-dichloro-2,4-(1*H*,3*H*,5*H*)-(1)-benzopyrano-(2,3-d)-pyrimidinedione,
8-methoxy-2,4-(1*H*,3*H*,5*H*)-(1)-benzopyrano-(2,3-d)-pyrimidinedione,
7,9-dibromo-2,4-(1*H*,3*H*,5*H*)-(1)-benzopyrano-(2,3-d)-pyrimidinedione,
8-trifluoromethyl-2,4-(1*H*,3*H*,5*H*)-(1)-benzopyrano-(2,3-d)-pyrimidinedione.

Example VIII

7-Carboxy-2,4-(1*H*,3*H*,5*H*)-(1)-Benzopyrano-(2,3-d)-Pyrimidinedione

Warm a suspension of 7 - methoxycarbonyl - 2,4 - (1*H*,3*H*,5*H*) - (1) - benzopyrano - (2,3 - d) - pyrimidinedione (5 g) in 1N-sodium hydroxide (25 ml) until a clear solution is formed and continue the warming for an additional $\frac{1}{2}$ hour. Filter the solution and acidify the filtrate with 2N sulfuric acid. Filter and wash the solids with water, isopropanol and then ether. Dry the washed solid to obtain 7 - carboxy - 2,4 - (1*H*,3*H*,5*H*) - (1) - benzopyrano - (2,3 - d) - pyrimidinedione $\cdot H_2O$; m.p. > 360°C. Also produced by this method are:
7-carboxy-3-methyl-2,4-(1*H*,3*H*,5*H*)-(1)-benzopyrano-(2,3-d)-pyrimidinedione,
7-carboxy-3-ethyl-2,4-(1*H*,3*H*,5*H*)-(1)-benzopyrano-(2,3-d)-pyrimidinedione,
7-carboxy-5-methyl-2,4-(1*H*,3*H*,5*H*)-(1)-benzopyrano-(2,3-d)-pyrimidinedione,
6-carboxy-2,4-(1*H*,3*H*,5*H*)-(1)-benzopyrano-(2,3-d)-pyrimidinedione,
8-carboxy-2,4-(1*H*,3*H*,5*H*)-(1)-benzopyrano-(2,3-d)-pyrimidinedione,
9-carboxy-2,4-(1*H*,3*H*,5*H*)-(1)-benzopyrano-(2,3-d)-pyrimidinedione,
7,8-dicarboxy-2,4-(1*H*,3*H*,5*H*)-(1)-benzopyrano-(2,3-d)-pyrimidinedione.

Example IX

7-(Isobutyryloxymethyl)-2,4-(1*H*,3*H*,5*H*)-(1)-Benzopyrano-(2,3-d)-Pyrimidinedione

To a cooled (below 5°C) suspension of 7 - hydroxymethyl - 2,4 - (1*H*,3*H*,5*H*) - (1) - benzopyrano - (2,3 - d) - pyrimidinedione. 1/3 $H_2O$ (2.56 g) in dry pyridine (100 ml), add isobutyric anhydride (20 ml). Keep the mixture cold for 24 hour and stir the mixture at 20°C for an additional two days. Pour the mixture over ice/water, filter and successively wash the solids with water, ethanol and ether. Dry the washed solids to obtain 7 - isobutyryloxymethyl) - 2,4 - (1*H*,3*H*,5*H*) - (1) - benzopyrano - (2,3 - d) - pyrimidinedione, m.p. 264.5—265°C.

Similarly, by starting withn other pyrimidinediones of this invention which bear one or more reactive hydroxyl groups (i.e., when R represents hydroxy or hydroxyalkyl), the foregoing esterification may be effected. Analogously, by utilizing the acid chloride or anhydride of the appropriate acid and following the techniques of this example, the following esters (of either the hydroxy or the hydroxyalkyl substituents) may be prepared: acetyl, propionyl, n-butyryl, isobutyryl, pivaloyl, t-butylacetyl, and lauroyl.

Additionally such esters are also formed from all the other compounds bearing a reactive hydroxy moiety as a substituent in the benzenoid moiety of the benzopyrano pyrimidinedione compounds.

Example X

7-(1′-Hydroxyethyl)-2,4-(1*H*,3*H*,5*H*)-(1)-Benzopyrano-(2,3-d)-Pyrimidinedione

To a stirred suspension of 7 - acetyl - 2,4 - (1*H*,3*H*,5*H*) - (1) - benzopyrano - (2,3 - d) - pyrimidinedione (0.5 g) in isopropanol (75 ml) add sodium borohydride (0.36 g) and continue stirring the resulting mixture at room temperature for $3\frac{1}{2}$ hours. Carefully add water and then add a saturated solution of ammonium chloride. Acidify the mixture with 5.5N hydrochloric acid, evaporate off the isopropanol, filter and wash the product with water and isopropanol to yield 7 - (1′ - hydroxyethyl) - 2,4 - (1*H*,3*H*,5*H*) - (1) - benzopyrano - (2,3 - d) - pyrimidinedione, m.p. 270—274°C.

Also, by the methodology of this example there may be produced the following compounds:
7-(1′-hydroxypropyl)-2,4-(1*H*,3*H*,5*H*)-(1)-benzopyrano-(2,3-d)-pyrimidinedione,
7-(1′-hydroxybutyl)-2,4-(1*H*,3*H*,5*H*)-(1)-benzopyrano-(2,3-d)-pyrimidinedione.

Example XI

7-(n-Pentyloxymethyl)-2,4-(1*H*,3*H*,5*H*)-(1)-Benzopyrano-(2,3-d)-Pyrimidinedione

Reflux (136—138°C) a mixture of 2.5 g of 7 - hydroxymethyl - 2,4 - (1*H*,3*H*,5*H*) - (1) - benzopyrano - (2,3 - d) - pyrimidinedione. 1/3 $H_2O$, 3.8 g of p-toluenesulfonic acid monohydrate and 250 ml of n-pentanol until the solution becomes homogeneous. Concentrated the solution until a solid begins to appear at which time the solution is cooled, diluted with ether (250) ml), filtered, triturated, and washed with fresh ether to yield 7 - (n - pentyloxymethyl) - 2,4 - (1*H*,3*H*,5*H*) - (1) - benzopyrano - (2,3 - d) - pyrimidinedione $\cdot$ 1/3 $H_2O$.

Also by substantially following the teachings of this example (except the reactants are heated to 130—140°C under pressure) there are produced the following compounds:
7-n-butyloxymethyl-2,4-(1*H*,3*H*,5*H*)-(1)-benzopyrano-(2,3-d)-pyrimidinedione,
7-iso-butyloxymethyl-2,4-(1*H*,3*H*,5*H*)-(1)-benzopyrano-(2,3-d)-pyrimidinedione,
7-n-propyloxymethyl-2,4-(1*H*,3*H*,5*H*)-(1)-benzopyrano-(2,3-d)-pyrimidinedione,
7-iso-propyloxymethyl-2,4-(1*H*,3*H*,5*H*)-(1)-benzopyrano-(2,3-d)-pyrimidinedione,
7-ethoxymethyl-2,4-(1*H*,3*H*,5*H*)-(1)-benzopyrano-(2,3-d)-pyrimidinedione,
7-methoxymethyl-2,4-(1*H*,3*H*,5*H*)-(1)-benzopyrano-(2,3-d)-pyrimidinedione.

Example XII
7-Bromomethyl-2,4-(1*H*,3*H*,5*H*)-(1)-Benzopyrano-(2,3-d)-Pyrimidinedione

To ice cold concentrated HBr (48 percent), (80 ml), add concentrated $H_2SO_4$ (10 ml) slowly and with continuous cooling. To this solution add 7 - hydroxymethyl - 2,4 - (1*H*,3*H*,5*H*) - (1) - benzopyrano - (2,3 - d) - pyrimidinedione · 1/2 $H_2O$ (10 g). With continuous stirring heat the mixture to 70—75°C and gradually add an additional 10 ml of concentrated $H_2SOl_4$. After three days pour the mixture into ice water, filter the mixture, wash the product with water and ethanol to yield 7 - bromomethyl - 2,4 - (1*H*,3*H*,5*H*) - (1) - benzopyrano - (2,3 - d) - pyrimidinedione.

Example XIII
7-Formyl-2,4-(1*H*,3*H*,5*H*)-(1)-Benzopyrano-(2,3-d)-Pyrimidinedione

Cool to —20°C a mixture containing 7 - hydroxymethyl - 2,4 - (1*H*,3*H*,5*H*) - (1) - benzopyrano - (2,3 - d) - pyrimidinedione · 1/3 $H_2O$. (10 g), dry dimethyl sulfoxide (60 ml) and hexamethylphosphoramide (100 ml) and to the cooled mixture add, in a portion-wise fashion, methanesulfonic anhydride (25 g) with continuous stirring. Allow the mixture to warm slightly for about one hour. Re-cool to —20°C slowly, and in a portion-wise fashion add triethylamine (45 g). After warming to room temperature pour the product into acidified water. Filter the product, wash with water, then isopropanol and finally with ether. Dry the product to yield 7 - formyl - 2,4 - (1*H*,3*H*,5*H*) - (1) - benzopyrano - (2,3 - d) - pyrimidinedione.

Example XIV
7-(n-Butylaminocarbonyl)-2,4-(1*H*,3*H*,5*H*)-(1)-Benzopyrano-(2,3-d)-Pyrimidinedione

7 - (imidazolylcarbonyl) - 2,4 - (1*H*,3*H*,5*H*) - (1) - benzopyrano - (2,3 - d) - pyrimidinedione (1 g) is suspended in dry dimethylformamide (60 ml) and n-butylamine (1.1 g) is added. The mixture is stirred at room temperature overnight then at 70°C for 1½ hours. The product is added to dilute HCl and the solid is collected, washed with water, ethanol and ether and dried to yield the desired 7 - (n - butylaminocarbonyl) - 2,4 - (1*H*,3*H*,5*H*) - (1) - benzopyrano - (2,3 - d) - pyrimidinedione, dec. 300°C.

Example XV
7-(5-Tetrazolylaminocarbonyl)-2,4-(1*H*,3*H*,5*H*)-(1)-Benzopyrano-(2,3-d)-Pyrimidinedione

To a suspension of 7 - carboxyl - 2,4 - (1*H*,3*H*,5*H*) - (1) - benzopyrano - (2,3 - d) - pyrimidinedione (2.46 g) in dry dimethylformamide (160 ml) at 75°C, add (all at once), N,N'-carbonyldiimidazole (3.05 g). Heat the resulting mixture at 68—73°C for 20 minutes, then add a solution of 5-aminotetrazole (0.80 g) in dry dimethylformamide (11 ml) at 40°C. Stir the resulting mixture overnight at room temperature and heat at 50°C for 24 hours. Chill the resulting mixture at 0°C for one day, filter, triturate with ether (25 ml), triturate with 20% v/v ethanol/ether, filter, wash with ether and vacuum dry at 54°C for two hours, to yield 7 - (5 - tetrazolylaminocarbonyl) - 2,4 - (1*H*,3*H*,5*H*) - (1) - benzopyrano - (2,3 - d) - pyrimidinedione, dec. 310°C.

Also, by the techniques illustrated by this and the previous example there are produced the following compounds: 7 - (2 - [2 - Pyridyl]ethylaminocarbonyl) - 2,4 - (1*H*,3*H*,5*H*) - (1) - benzopyrano - (2,3 - d) - pyrimidinedione, dec. 295°C, 3 - methyl - 7 - (2 - [4 - pyridyl]ethylaminocarbonyl) - 2,4 - (1*H*,3*H*,5*H*) - (1) - benzopyrano - (2,3 - d) - pyrimidinedione.

5 - methyl - 7 - (2 - [3 - pyridyl]ethylaminocarbonyl) - 2,4 - (1*H*,3*H*,5*H*) - (1) - benzopyrano - (2,3 - d) - pyrimidinedione,

8 - (3 - [2 - pyridyl]propylaminocarbonyl) - 2,4 - (1*H*,3*H*,5*H*) - (1) - benzpyrano - (2,3 - d) - pyrimidinedione,

7 - (2 - pyridylmethylaminocarbonyl) - 2,4 - (1*H*,3*H*,5*H*) - (1) - benzopyrano - (2,3 - d) - pyrimidinedione,

8 - (3 - pyridylmethylaminocarbonyl) - 2,4 - (1*H*,3*H*,5*H*) - (1) - benzopyrano - (2,3 - d) - pyrimidinedione,

7 - (4 - pyridylmethylaminocarbonyl) - 2,4 - (1*H*,3*H*,5*H*) - (1) - benzopyrano - (2,3 - d) - pyrimidinedione,

7 - (6' - methyl - 2 - pyridylethylaminocarbonyl) - 2,4 - (1*H*,3*H*,5*H*) - (1) - benzopyrano - (2,3 - d) - pyrimidinedione,

7 - (2 - hydroxy - 2 - phenylethylaminocarbonyl) - 2,4 - (1*H*,3*H*,5*H*) - (1) - benzopyrano - (2,3 - d) - pyrimidinedione,

7 - [(1'R,2'S) - (1' - hydroxy - 1' - phenylprop - 2' - yl)aminocarbonyl] - 2,4 - (1*H*,3*H*,5*H*) - (1) - benzopyrano - (2,3 - d) - pyrimidinedione, m.p. 276—280°C,

7 - (2 - hydroxy - 2 - [2 - pyridyl]ethylaminocarbonyl) - 24 - (1*H*,3*H*,5*H*) - (1) - benzopyrano - (2,3 - d) - pyrimidinedione,
8 - (2 - hydroxy - 2[2 - pyridyl] ethylaminocarbonyl) - 2,4 - (1*H*,3*H*,5*H*) - (1) - benzopyrano - (2,3 - d) - pyrimidinedione,
7 - (2 - hydroxy - 2 - phenyl - 2 - [2 - pyridyl]ethylaminocarbonyl) - 2,4 - (1*H*,3*H*,5*H*) - (1) - benzo - pyrano - (2,3 - d) - pyrimidinedione,
7 - (2 - hydroxy - 2 - phenyl - 2 - [3 - pyridyl]ethylaminocarbonyl) - 2,4 - (1*H*,3*H*,5*H*) - (1) - benzo - pyrano - (2,3 - d) - pyrimidinedione,
7 - (2 - hydroxy - 2 - phenyl - 2 - [6 - methyl - 2 - pyridyl]ethylaminocarbonyl) - 2,4 - (1*H*,3*H*,5*H*) - (1) - benzopyrano - (2,3 - d) - pyrimidinedione,
7 - (methoxyethylaminocarbonyl) - 2,4 - (1*H*,3*H*,5*H*) - (1) - benzopyrano - (2,3 - d) - pyrimidinedione, dec. 320°C,
7 - (2′ - hydroxyethylaminocarbonyl) - 2,4 - (1*H*,3*H*,5*H*) - (1) - benzopyrano - (2,3 - d) - pyrimidinedione, chars 325°C,
7 - (3′ - hydroxypropylaminocarbonyl) - 2,4 - (1*H*,3*H*,5*H*) - (1) - benzopyrano - (2,3 - d) - pyrimidinedione,
7 - (2′,2′,2′ - trifluoroethylaminocarbonyl) - 2,4 - (1*H*,3*H*,5*H*) - (1) - benzopyrano - (2,3 - d) - pyrimidinedione, m.p. > 325°C,
7 - (3′,3′,3′ - trifluoropropylaminocarbonyl) - 2,4 - (1*H*,3*H*,5*H*) - (1) - benzopyrano - (2,3 - d) - pyrimidinedione,
7 - (2′,2′,3′,3′,3′ - pentafluoropropylaminocarbonyl) - 2,4 - (1*H*,3*H*,5*H*) - (1) - benzopyrano - (2,3 - d) - pyrimidinedione,
7 - (dimethylaminocarbonyl) - 2,4 - (1*H*,3*H*,5*H*) - (1) - benzopyrano - (2,3 - d) - pyrimidinedione,
7 - (diethylaminocarbonyl) - 2,4 - (1*H*,3*H*,5*H*) - (1) - benzopyrano - (2,3 - d) - pyrimidinedione,
7 - (morpholinocarbonyl) - 2,4 - (1*H*,3*H*,5*H*) - (1) - benzopyrano - (2,3 - d) - pyrimidinedione,
7 - (pyrrolidinocarbonyl) - 2,4 - (1*H*,3*H*,5*H*) - (1) - benzopyrano - (2,3 - d) - pyrimidinedione,
7 - (piperidinocarbonyl) - 2,4 - (1*H*,3*H*,5*H*) - (1) - benzopyrano - (2,3 - d) - pyrimidinedione, m.p. > 300°C,
7 - (N′ - methylpiperazinylcarbonyl) - 2,4 - (1*H*,3*H*,5*H*) - (1) - benzopyrano - (2,3 - d) - pyrimidinedione,
7 - (N′ - hydroxyethylpiperazinylcarbonyl) - 2,4 - (1*H*,3*H*,5*H*) - (1) - benzopyrano - (2,3 - d) - pyrimidinedione,
7 - (diallylaminocarbonyl) - 2,4 - (1*H*,3*H*,5*H*) - (1) - benzopyrano - (2,3 - d) - pyrimidinedione, dec. 205°C,
7 - (methylthioethylaminocarbonyl) - 2,4 - (1*H*,3*H*,5*H*) - (1) - benzopyrano - (2,3 - d) - pyrimidinedione,
7 - (methylsulfoxyethylaminocarbonyl) - 2,4 - (1*H*,3*H*,5*H*) - (1) - benzopyrano - (2,3 - d) - pyrimidinedione,
7 - (methylsulfonylethylaminocarbonyl) - 2,4 - (1*H*,3*H*,5*H*) - (1) - benzopyrano - (2,3 - d) - pyrimidinedione,
7 - (2 - thiazolylaminocarbonyl) - 2,4 - (1*H*,3*H*,5*H*) - (1) - benzopyrano - (2,3 - d) - pyrimidinedione, m.p. > 325°C,
7 - (2 - oxazolylaminocarbonyl) - 2,4 - (1*H*,3*H*,5*H*) - (1) - benzopyrano - (2,3 - d) - pyrimidinedione,
7 - (2 - (1,3,4 - thiadiazolyl) - aminocarbonyl) - 2,4 - (1*H*,3*H*,5*H*) - (1) - benzopyrano - (2,3 - d) - pyrimidinedione,
7 - (2 - (5 - methyl - 1,3,4 - thiadiazolyl - 2,4 - (1*H*,3*H*,5*H*) - (1) - benzopyrano - (2,3 - d) - pyrimidinedione,
8 - ethylaminocarbonyl - 2,4 - (1*H*,3*H*,5*H*) - (1) - benzopyrano - (2,3 - d) - pyrimidinedione,
8 - *n* - propylaminocarbonyl - 2,4 - (1*H*,3*H*,5*H*) - (1) - benzopyrano - (2,3 - d) - pyrimidinedione,
9 - bromo - 7 - *n* - butylaminocarbonyl - 2,4 - (1*H*,3*H*,5*H*) - (1) - benzopyrano - (2,3 - d) - pyrimidinedione, chars 300°C,
8 - iso - butylaminocarbonyl - 2,4 - (1*H*,3*H*,5*H*) - (1) - benzopyrano - (2,3 - d) - pyrimidinedione,
8 - *n* - pentylaminocarbonyl - 2,4 - (1*H*,3*H*,5*H*) - (1) - benzopyrano - (2,3 - d) - pyrimidinedione,
7 - (5 - (1,2,4 - thiadiazolyl)aminocarbonyl) - 2,4 - (1*H*,3*H*,5*H*) - (1) - benzopyrano - (2,3 - d) - pyrimidinedione,
7 - (5 - (3 - methyl - 1,2,4 - thiadiazolyl) - 2,4 - (1*H*,3*H*,5*H*) - (1) - benzopyrano - (2,3 - d) - pyrimidinedione,
7 - (2 - furylaminocarbonyl) - 2,4 - (1*H*,3*H*,5*H*) - (1) - benzopyrano - (2,3 - d) - pyrimidinedione,
7 - (3 - pyrazolylaminocarbonyl) - 2,4 - (1*H*,3*H*,5*H*) - (1) - benzopyrano - (2,3 - d) - pyrimidinedione,
7 - (2 - methyl - 5 - tetrazolylaminocarbonyl) - 2,4 - (1*H*,3*H*,5*H*) - (1) - benzopyrano - (2,3 - d) - pyrimidinedione,
7 - (*n* - butylaminocarbonyl) - 2,4 - (1*H*,3*H*,5*H*) - (1) - benzopyrano - (2,3 - d) - pyrimidinedione,
7 - methylaminocarbonyl - 2,4 - (1*H*,3*H*,5*H*) - (1) - benzopyrano - (2,3 - d) - pyrimidinedione,

7 - ethylaminocarbonyl - 2,4 - (1*H*,3*H*,5*H*) - (1) - benzopyrano - (2,3 - d) - pyrimidinedione,
7 - *n* - propylaminocarbonyl - 2,4 - (1*H*,3*H*,5*H*) - (1) - benzopyrano - (2,3 - d) - pyrimidinedione, dec. 278°C,
7 - isopropylaminocarbonyl - 2,4 - (1*H*,3*H*,5*H*) - (1) - benzopyrano - (2,3 - d) - pyrimidinedione,
7 - isobutylaminocarbonyl - 2,4 - (1*H*,3*H*,5*H*) - (1) - benzopyrano - (2,3 - d) - pyrimidinedione,
7 - sec - butylaminocarbonyl - 2,4 - (1*H*,3*H*,5*H*) - (1) - benzopyrano - (2,3 - d) - pyrimidinedione,
7 - tert - butylaminocarbonyl - 2,4 - (1*H*,3*H*,5*H*) - (1) - benzopyrano - (2,3 - d) - pyrimidinedione,
7 - *n* - pentylaminocarbonyl - 2,4 - (1*H*,3*H*,5*H*) - (1) - benzopyrano - (2,3 - d) - pyrimidinedione, chars 300°C,
7 - *n* - hexylaminocarbonyl - 2,4 - (1*H*,3*H*,5*H*) - (1) - benzopyrano - (2,3 - d) - pyrimidinedione,
7 - *n* octylaminocarbonyl - 2,4 - (1*H*,3*H*,5*H*) - (1) - benzopyrano - (2,3 - d) - pyrimidinedione,
7 - *n* - decylaminocarbonyl - 2,4 - (1*H*,3*H*,5*H*) - (1) - benzopyrano - (2,3 - d) - pyrimidinedione, m.p. > 300°C,
7 - benzylaminocarbonyl - 2,4 - (1*H*,3*H*,5*H*) - (1) - benzopyrano - (2,3 - d) - pyrimidinedione,
7 - benzhydryl - aminocarbonyl - 2,4 - (1*H*,3*H*,5*H*) - (1) - benzopyrano - (2,3 - d) - pyrimidinedione,
7 - cyclopropylaminocarbonyl - 2,4 - (1*H*,3*H*,5*H*) - (1) - benzopyrano - (2,3 - d) - pyrimidinedione,
7 - cyclobutylaminocarbonyl - 2,4 - (1*H*,3*H*,5*H*) - (1) - benzopyrano - (2,3 - d) - pyrimidinedione,
7 - cyclopentylaminocarbonyl - 2,4 - (1*H*,3*H*,5*H*) - (1) - benzopyrano - (2,3 - d) - pyrimidinedione,
7 - cyclohexylaminocarbonyl - 2,4 - (1*H*,3*H*,5*H*) - (1) - benzopyrano - (2,3 - d) - pyrimidinedione, m.p. > 300°C,
7 - cycloheptylaminocarbonyl - 2,4 - (1*H*,3*H*,5*H*) - (1) - benzopyrano - (2,3 - d) - pyrimidinedione,
7 - cyclopropylmethylaminocarbonyl - 2,4 - (1*H*,3*H*,5*H*) - (1) - benzopyrano - (2,3 - d) - pyrimidinedione,
7 - cyclobutylmethylaminocarbonyl - 2,4 - (1*H*,3*H*,5*H*) - (1) - benzopyrano - (2,3 - d) - pyrimidinedione,
7 - (4 - hydroxy - 2 - pyrimidinylaminocarbonyl) - 2,4 - (1*H*,3*H*,5*H*) - (1) - benzopyrano - (2,3 - d) - pyrimidinedione,
7 - (2,4 - dihydroxy - 5 - pyrimidinylaminocarbonyl) - 2,4 - (1*H*,3*H*,5*H*) - (1) - benzopyrano - (2,3 - d) - pyrimidinedione,
7 - (5 - (1,3 - dimethylpyrimidin - 2,4 - dionyl)aminocarbonyl) - 2,4 - (1*H*,3*H*,5*H*) - (1) - benzopyrano - (2,3 - d) - pyrimidinedione,
7 - (2 - [3 - pyridyl]ethylaminocarbonyl) - 2,4 - (1*H*,3*H*,5*H*) - (1) - benzopyrano - (2,3 - d) - pyrimidinedione,
7 - (2 - [4 - pyridyl] - ethylaminocarbonyl) - 2,4 - (1*H*,3*H*,5*H*) - (1) - benzopyrano - (2,3 - d) - pyrimidinedione,
7 - (2 - [*N* - piperidinyl]ethylaminocarbonyl) - 2,4 - (1*H*,3*H*,5*H*) - (1) - benzopyrano - (2,3 - d) - pyrimidinedione,
7 - (2 - [2 - pyridyl]propylaminocarbonyl) - 2,4 - (1*H*,3*H*,5*H*) - (1) - benzopyrano - (2,3 - d) - pyrimidinedione,
7 - (3 - [2 - pyridyl]propylaminocarbonyl) - 2,4 - (1*H*,3*H*,5*H*) - (1) - benzopyrano - (2,3 - d) - pyrimidinedione,
7 - (3 - [3 - pyridyl]propylaminocarbonyl) - 2,4 - (1*H*,3*H*,5*H*) - (1) - benzopyrano - (2,3 - d) - pyrimidinedione,
7 - (3 - [4 - pyridyl]propylaminocarbonyl) - 2,4 - (1*H*,3*H*,5*H*) - (1) - benzopyrano - (2,3 - d) - pyrimidinedione,
7 - (6 - methyl - 2 - pyridylmethylaminocarbonyl) - 2,4 - (1*H*,3*H*,5*H*) - (1) - benzopyrano - (2,3 - d) - pyrimidinedione,
7 - (2 - phenyl - [2 - pyridyl] - ethylaminocarbonyl) - 2,4 - (1*H*,3*H*,5*H*) - (1) - benzopyrano - (2,3 - d) - pyrimidinedione,
7 - (2 - phenyl - 2 - [3 - pyridyl] - ethylaminocarbonyl) - 2,4 - (1*H*,3*H*,5*H*) - (1) - benzopyrano - (2,3 - d) - pyrimidinedione,
7 - (2 - phenyl - 2 - [4 - pyridyl] - ethylaminocarbonyl) - 2,4 - (1*H*,3*H*,5*H*) - (1) - benzopyrano - (2,3 - d) - pyrimidinedione,
7 - (3 - phenyl - 3 - [2 - pyridyl]propylaminocarbonyl) - 2,4 - (1*H*,3*H*,5*H*) - (1) - benzopyrano - (2,3 - d) - pyrimidinedione,
7 - (3 - phenyl - 3 - [3 - pyridyl]propylaminocarbonyl) - 2,4 - (1*H*,3*H*,5*H*) - (1) - benzopyrano - (2,3 - d) - pyrimidinedione,
7 - (3 - phenyl - 3 - [4 - pyridyl]propylaminocarbonyl) - 2,4 - (1*H*,3*H*,5*H*) - (1) - benzopyrano - (2,3 - d) - pyrimidinedione,
7 - (3 - phenyl - 3 - [6 - methyl - 2 - pyridyl]propylaminocarbonyl) - 2,4 - (1*H*,3*H*,5*H*) - (1) - benzopyrano - (2,3 - d) - pyrimidinedione,
7 - (2,2 - diphenylethylaminocarbonyl) - 2,4 - (1*H*,3*H*,5*H*) - (1) - benzopyrano - (2,3 - d) - pyrimidinedione,

7 - (2 - phenylethylaminocarbonyl) - 2,4 - (1*H*,3*H*,5*H*) - (1) - benzopyrano - (2,3 - d) - pyrimidinedione,
7 - (2 - [2 - pyridyl] - ethyl - *N* - methylaminocarbonyl) - 2,4 - (1*H*,3*H*,5*H*) - (1) - benzopyrano - (2,3 - d) - pyrimidinedione,
2′ - [7 - (2,4 - (1*H*,3*H*,5*H*) - (1) - benzopyrano - (2,3 - d) - pyrimidinedionyl)-carboxamido]acetamide.

Example XVI
7-Imidazolylcarbonyl-2,4-(1*H*,3*H*,5*H*)-(1)-Benzopyrano-(2,3-d)-Pyrimidinedione

Suspend 7 - carboxy - 2,4 - (1*H*,3*H*,5*H*) - (1) - benzopyrano - (2,3 - d) - pyrimidinedione (57 g) in dry dimethylformamide at 40°C. Add carbonyldiimidazole (114 g) in portions, waiting until the reaction subsides before adding the next portion. After all has been added, warm the mixture to 70°C for 3 hours then allow to stand at room temperature overnight. filter off the solid. Wash with isopropanol and ether consecutively, and dry to yield 7 - imidazolylcarbonyl - 2,4 - (1*H*,3*H*,5*H*) - (1) - benzopyrano - (2,3 - d) - pyrimidinedione.

The compounds of the invention (I) have the applied use characteristic of being anti-allergic agents useful in the prophylactic treatment of sensitized animals for allergy and anaphylactic reaction of a reagin or non-reagin mediated nature and as such are useful in the treatment of such disease states as all forms of asthma, allergic rhinitis, and any allergic condition induced by antibodies.

In testing the compounds of this invention, the two primary assays utilized are the Passive Cutaneous Anaphylaxis (PCA) assay and the Antigen induced histamine release from rat peritoneal mast cells passively sensitized in vivo assay. The compounds are additionally tested as antagonists of intradermally injected histamine and serotonin; these secondary tests show that the compounds are not working as classical antihistamine or anti-serotonin anti-allergy agents.

These tests may be briefly described as follows: Preparation of Antisera Containing Homocytotropic Antibody (IgE) to N. Brasiliensis: Male Sprague-Dawley rats, 150—200 g, were injected subcutaneously with 3000 larvae and 28 days later were reinfected with 3000 larvae. Serum was collected 7—12 days following reinfection and frozen in small aliquots at —40°C.

Preparation of Antigen: Adult worms were harvested from the small intestine of rats 8—11 days after infection with larvae. A homogenate was prepared from a suspension of worms in 0.15 M saline using a ground glass homogenizer. The homogenate was centrifuged at 3000 × g for 15 minutes in a Sorvall RC2-B centrifuge. The supernatant was carefully removed and frozen at —40°C. Just prior to use as antigen, the supernatant was adjusted to a concentration of 5 mg protein per milliliter. All antigen preparations were standardized in this way although it is recognized that the protein concentration of this crude worm extract does not necessarily reflect the amount of specific antigen present in the extract.

Passive cutaneous Anaphylaxis (PCA): Male Sprague-Dawley rats weighing 250—300 g were used for PCA reactions. Nine two-fold dilutions of antisera containing homocytotropic antibodies against N. brasiliensis were made in 0.15 M saline. Each dilution was injected intradermally at separate sites onto the shaved backs of normal rats and 48 hours later the animals were challenged intravenously with 0.1 ml antigen (worm extract) mixed with 0.9 ml of one percent Evans blue dye. The animals were sacrificed 45 minutes following antigen challenge, skinned, and the area of blueing measured with a millimeter rule. The diameter of the sites of reaction were graded as follows:

| Diameter | Score |
|---|---|
| 20 (or greater) | 4 |
| 15—19 | 3 |
| 14—10 | 2 |
| 5—9 | 1 |

The intensity of the reaction was also graded from 0—4.

Histamine Release from Rat Peritoneal Mast Cells Passively Sensitized in vivo: The method used in these studies is a modification of that of Orange, et al. Briefly, male Sprague-Dawley rats (CD strain), 150—200 g were injected i.p. with 2.0 ml of a dilution of rat antisera containing HA. Two hours later the animals were challenged with 100 mg worm protein in 5.0 ml. Tyrode's solution containing 50 mg/ml of heparin. Exactly five minutes later, the peritoneal fluid was harvested and centrifuged at 150 × g for five minutes at 4°C. The supernatants were removed, and the cells were resuspended in 1.0 ml. Tyrode's and boiled for seven minutes to extract residual cell histamine. The supernatant and cell extracts were frozen at —70°C and later assayed for their histamine content.

Histamine Release in vitro from Passively Sensitized Mast Cells: Peritoneal mast cells were obtained from normal animals, pooled, washed and resuspended in Tyrode's minus gelatin buffer. An equal volume of serum containing rat homocytotropic antibody was added to the cell suspension and the mixture was incubated at 37°C for two hours in a metabolic shaker. The suspension was then centrifuged at 150 x g for 10 minutes and the supernatant discarded. The cells were resuspended in Tyrode's minus gelatin, combined with antigen, and incubated for five minutes at 37°C. The cells were harvested by centrifugation and the supernatants immediately frozen at —70°C. Residual histamine was extracted from the cells by boiling them for seven minutes in 1.0 ml Tyrode's minus gelatin.

Histamine Release in vitro from Actively Sensitized Mast Cells: Peritoneal mast cells were obtained from rats 21—28 days following infection with 3000 Nippostrongylus (NB) larvae. The cells were washed and suspended in Tyrode's buffer. Inhibitor dissolved in Tyrode's was added one minute before antigen. An equal volume of antigen, prewarmed to 37°C, was added and the mixture incubated at 37°C for 15 minutes. The cells and supernatant were then processed as described above for passive sensitization. Both in vitro methods are essentially those described by Wilson, et al.

Histamine Assay: The fluorescent assay of Shore, et al as modified by Technicon for automated determination of histamine was employed.

Determination of Antihistamine and Antiserotonin Activities of Compounds: Normal rats, 200—250 g, were injected i.d. with 10, 20 and 50 mg of histamine and/or serotonin at separate sites onto their backs 30 or 60 minutes following i.p. or oral administration of drug, respectively. Immediately following the last i.d. injection the animals received 1 ml of a one percent solution of Evans blue dye intravenously. Fifteen minutes later they were sacrificed, and the area of blueing measured with a millimeter rule. Each compound was tested for antihistamine or antiserotonin activity in nine animals.

From these tests, as well as by comparison with known anti-allergy agents of similar type activity, i.e., Intal) it has been found that the compounds are effective in the treatment of the above mentioned allergic disease states and that such activity is not a function of the classical antihistamine and antiserotonin characteristics. The compounds are effective for their end-use at varying dose ranges, depending on the method of administration. For example, the more active compounds have an effective interperitoneal administered dose of 0.1—10 MPK, an effective intravenous administered dose of 0.01—10 MPK, and an effective oral dosage of 10—200 MPK (MPK ... milligram per kilogram of body-weight). In general, the compounds of this invention, when compared with the clinical effectiveness of Intal-like compounds in aerosol or inhalation preparations are effective at 1—20 mg per day.

In their use as anti-allergy agents, the compounds of this invention are administered in unit dosage forms, such as tablets, capsules, pills, powders, granules, sterile parenteral solutions or suspensions, eye drops, oral solutions or suspensions, and oil-in-water and water-in-oil emulsions containing suitable quantities of the compounds of this invention (I).

For oral administration, either solid or fluid unit dosage forms can be prepared. For preparing solid compositions such as tablets, the compounds of this invention (I) are mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by mixing the compound with an inert pharmaceutical diluent and filling the mixture into a hard gelatin capsule of appropriate size. Soft gelatine capsules are prepared by machine encapsulation of a slurry of the compound with an acceptable vegtable oil, light liquid petrolatum, or other inert oil.

Fluid unit dosage forms for oral administration such as syrups, elixirs and suspensions can be prepared. The water soluble forms can be dissolved in an aqueous vehicle together with sugar, aromatic flavoring agents and preservatives to form a syrup. An elixir is prepared by using an alcoholic (ethanol) vehicle with suitable sweeteners such as sugar and saccharin, together with an aromatic flavoring agent.

Suspensions can be prepared with an aqueous vehicle with the aid of a suspending agent such as acacia, tragacanth, methylcellulose and the like.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampul and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Additionally, a rectal suppository can be employed to deliver the active compound. This dosage form is of particular interest where the mammal cannot be treated conveniently by means of other dosage forms, such as orally or by insufflation, as in the case of young children or debilitated persons. The active compound can be incorporated into any of the known suppository bases by methods known in the art. Examples of such bases include cocoa butter, polyethylene glycols (Carbowaxes), polyethylene sorbitan monostearate, and mixtures of these with other compatible materials to modify the melting point or dissolution rate. These rectal suppositories can weigh from about 1—2.5 g.

For treatment of allergic conditions of the nose, such as rhinitis, compositions adapted for contact with nasal linings are preferred.

Compositions for inhalation are of three basic types: (1) a powder mixture preferably micropulverized with particle size, preferably from about 1 to about 5 microns; (2) an aqueous solution or suspension to be sprayed with a nebulizer; and (3) an aerosol with volatile propellant in a pressurized container.

The powders are quite simply prepared by mixing a compound of the formula with a solid base which is compatible with lung tissue, preferably lactose. The powders are packaged in a device adapted to emit a measured amount of powder when inhaled through the mouth.

Aqueous solutions are prepared by dissolving the compounds of this invention in water and adding salt to provide an isotonic solution and buffering to a pH compatible with inhalation. The solutions are dispersed in a spray device or nebulizer and sprayed into the mouth while inhaling.

Aerosols are prepared by dispersing a compound of the compounds of this invention (I) in water or ethanol, and mixing with a volatile propellant and placing in a pressurized contained having a metering valve to release a predetermined amount of material.

The following are specific examples of effective pharmaceutical formulations by which the compounds of this invention may be administered.

Formulation I

A lot of 10,000 tablets, each containing 1 mg of 7 - (5 - tetrazoylaminocarbonyl) - 2,4 - (1*H*,3*H*,5*H*) - (1) - benzopyrano - (2,3 - d) - pyrimidinedione is prepared from the following types and amounts of ingredients:

| | |
|---|---|
| 7-(5-tetrazolylaminocarbonyl)-2,4-(1*H*,3*H*,5*H*)-(1)-benzopyrano-(2,3-d)-pyrimidinedione | 10 g |
| Dicalcium phosphate | 1000 g |
| Methylcellulose U.S.P. (15 cps) | 60 g |
| Talc | 150 g |
| Corn starch | 200 g |
| Magnesium stearate | 10 g |

The compound and dicalcium phosphate are mixed well, granulated with 7.5 percent solution of methylcellulose in water, passed through a No. 8 screen and dried carefully. The dried granules are passed through a No. 12 screen, mixed thoroughly with talc, starch and magnesium stearate, and compressed into tablets.

Formulation II

One thousand tablets, each containing 50 mg of 7 - (5 - tetrazolylaminocarbonyl) - 2,4 - (1*H*,3*H*,5*H*) - (1) - benzopyrano - (2,3 - d) - pyrimidinedione are prepared from the following types and amounts of ingredients:

| | |
|---|---|
| 7-(5-tetrazolylaminocarbonyl)-2,4-(1*H*,3*H*,5*H*)-(1)-benzopyrano-(2,3-d)-pyrimidinedione | 50 g |
| Microcrystalline cellulose, NF | 410 g |
| Starch | 100 g |
| Magnesium stearate powder | 3 g |

The ingredients are screened and blended together and pressed into tablets.

Formulation III

A sterile preparation suitable for intramuscular injection and containing 5 mg of 7 - (5 - tetrazolylaminocarbonyl) - 2,4 - (1*H*,3*H*,5*H*) - (1) - benzopyrano - (2,3 - d) - pyrimidinedione in each milliliter is prepared from the following ingredients:

| | |
|---|---|
| 7-(5-tetrazolylaminocarbonyl)-2,4-(1*H*,3*H*,5*H*)-(1)-benzopyrano-(2,3-d)-pyrimidinedione | 5 g |
| Benzyl benzoate | 200 g |
| Methylparaben | 1.5 gm |
| Propylparaben | 0.5 g |
| Cottonseed oil q.s. | 1000 ml |

Formulation IV

Six hundred ml of an aqueous suspension containing 5.0 mg of the 7 - (5 - tetrazolylaminocarbonyl) - 2,4 - (1*H*,3*H*,5*H*) - (1) - benzopyrano - (2,3 - d) - pyrimidinedione per ml is prepared as follows:

| | |
|---|---|
| 7-(5-tetrazolylaminocarbonyl)-2,4-(1*H*,3*H*,5*H*)-(1)-benzopyrano-(2,3-d)-pyrimidinedione | 3.0 g |
| Sodium chloride | 5.0 g |
| Water for injection q.s. | 600 ml |

The 7 - (5 - tetrazolylaminocarbonyl) - 2,4 - (1*H*,3*H*,5*H*) - (1) - benzopyrano - (2,3 - d) - pyrimidinedione and sodium chloride are dispersed in sufficient water to make 600 ml and sterilized.

The liquid is placed in nebulizers designed to deliver 0.25 ml per spray.

Formulation V

A powder mixture consisting of 0.1 g of 7 - (5 - tetrazolylaminocarboinyl) - 2,4 - (1*H*,3,5*H*) - (1) - benzopyrano - (2,3 - d) - pyrimidinedione and sufficient lactose to make 5 g of mixture is micropulverized and placed in an insufflator designed to deliver 50 mg of powder per dose.

The powder is inhaled into the lungs every 4 to 6 hours for prevention of asthmatic attacks.

The powder is inhaled intranasally every 4 hours for prevention of rhinitis.

Formulation VI

One thousand tablets, each containing 5 mg of 7 - [2 - (2 - pyridyl)ethylaminocarbonyl] - 2,4 - (1*H*,3*H*,5*H*) - (1) - benzopyrano - (2,3 - d) - pyrimidinedione hydrochloride are prepared from the following types and amounts of ingredients:

| | |
|---|---|
| 7-[2-(2-pyridyl)ethylaminocarbonyl)-2,4-(1*H*,3*H*,5*H*)-(1)-benzopyrano-(2,3-d)-pyrimidinedione hydrochloride | 5 g |
| Microcrystalline cellulose NF | 410 g |
| Starch | 100 g |
| Magnesium stearate powder | 3 g |

The ingredients are screened and blended together and pressed into tablets.

As is true for most generic classes of compounds suitable for use as therapeutic agents, certain subgeneric and certain specific agents have a better biological profile than others. In this particular instance, those compounds having a carboxamido radical at the 7- or 8-positions, or a acyloxyalkyl radical at the 7- or 8-positions, or an alkoxycarbonyl radical at the 7- or 8-positions are most preferred. Preferably the 3- and/or the 5-positions are unsubstituted, but methyl radicals at one or the other

position when "A" represents a carboxamido, acyloxyalkyl or alkoxycarbonyl radical are advantageously employed. Specifically, desirable compounds are:

7 - isopropyloxycarbonyl - 2,4 - (1*H*,3*H*,5*H*) - (1) - benzopyrano - (2,3 - d) - pyrimidinedione,
7 - (*n* - butyryloxymethyl) - 2,4 - (1*H*,3*H*,5*H*) - (1) - benzopyrano - (2,3 - d) - pyrimidinedione,
7 - (isobutyryloxymethyl) - 2,4 - (1*H*,3*H*,5*H*) - (1) - benzopyrano - (2,3 - d) - pyrimidinedione,
7 - (isovaleryloxymethyl) - 2,4 - (1*H*,3*H*,5*H*) - (1) - benzopyrano - (2,3 - d) - pyrimidinedione,
7 - (*n* - propylaminocarbonyl) - 2,4 - (1*H*,3*H*,5*H*) - (1) - benzopyrano - (2,3 - d) - pyrimidinedione,
7 - (isopropylaminocarbonyl) - 2,4 - (1*H*,3*H*,5*H*) - (1) - benzopyrano - (2,3 - d) - pyrimidinedione,
7 - (*n* - butylaminocarbonyl - 2,4 - (1*H*,3*H*,5*H*) - (1) - benzopyrano - (2,3 - d) - pyrimidinedione,
7 - (isobutylaminocarbonyl) - 2,4 - (1*H*,3*H*,5*H*) - (1) - benzopyrano - (2,3 - d) - pyrimidinedione,
7 - (2 - hydroxyethylaminocarbonyl) - 2,4 - (1*H*,3*H*,5*H*) - (1) - benzopyrano - (2,3 - d) - pyrimidinedione,
7 - (tris - hydroxymethyl - methylaminocarbonyl) - 2,4 - (1*H*,3*H*,5*H*) - (1) - benzopyrano - (2,3 - d) - pyrimidinedione,
7 - (2 - hydroxy - 2 - phenylethylaminocarbonyl) - 2,4 - (1*H*,3*H*,5*H*) - (1) - benzopyrano - (2,3 - d) - pyrimidinedione,
7 - (2 hydroxy - 2 - (2 - pyridyl)ethylaminocarbonyl) - 2,4 - (1*H*,3*H*,5*H*) - (1) - benzopyrano - (2,3 - d) - pyrimidinedione,
7 - (2 - pyridylmethylaminocarbonyl) - 2,4 - (1*H*,3*H*,5*H*) - (1) - benzopyrano - (2,3 - d) - pyrimidinedione,
7 - (3 - (2 - pyridyl) - propylaminocarbonyl) - 2,4 - (1*H*,3*H*,5*H*) - (1) - benzopyrano - (2,3 - d) - pyrimidinedione,
3 - methyl - 7 - (5 - tetrazolylaminocarbonyl - 2,4 - (1*H*,3*H*,5*H*) - (1) - benzopyrano - (2,3 - d) - pyrimidinedione,
3 - methyl - 7 - (2 - [2 - pyridyl]ethylaminocarbonyl) - 2,4 - (1*H*,3*H*,5*H*) - (1) - benzopyrano - (2,3 - d) - pyrimidinedione,
7 - (5 - tetrazolylaminocarbonyl) - 2,4 - (1*H*,3*H*,5*H*) - (1) - benzopyrano - (2,3 - d) - pyrimidinedione,
7 - [2 - (2 - pyridyl)ethylaminocarbonyl] - 2,4 - (1*H*,3*H*,5*H*) - (1) - benzopyrano - (2,3 - d) - pyrimidinedione.

**Claims for the Contracting States: BE, CH, DE, FR, GB, IT, NL, SE**

1. Compounds of the formula

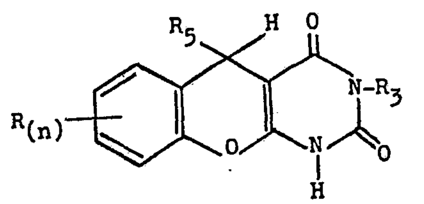

(I)

wherein each of $R_3$ and $R_5$ represents hydrogen or loweralkyl, n is an integer from one to four and R represents hydrogen loweralkyl, lowercycloalkyl, lowercycloalkylloweralkyl, acyloxyloweralkyl, hydroxyloweralkyl, loweralkoxyloweralkyl, nitro, halogeno, haloloweralkyl, hydroxy, loweralkoxy, acylaminoloweralkyl, aminoloweralkyl, mono- or diloweralkylaminoloweralkyl, alkanoyloxy, carboxy, loweralkoxycarbonyl, acyl, formyl, cyano or a carboxamido moiety of the structure

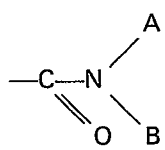

wherein A is straight or branched chain alkyl with up to 12 carbon atoms, lowercycloalkyl, lowercycloalkylloweralkyl, loweralkoxylloweralkyl, hydroxyloweralkyl, fluoroloweralkyl, loweralkenyl, loweralkylthioloweralkyl, loweralkylsulfoxyloweralkyl, loweralkylsulfonylloweralkyl, thiazolyl, oxazolyl, thiadiazolyl, methylthiadiazolyl, furyl, pyrazolyl, tetrazolyl, methyltetrazolyl, hydroxypyrimidinyl, phenyl, pyrimidinyl-dione, or the grouping

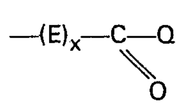

wherein E is a straight or branched chain loweralkylene or cyclic loweralkylene, X is zero or one and Q is a hydroxy, loweralkoxy, amino or mono- or diloweralkylamino; or the grouping E—$R_8$, wherein E is as defined above and is optionally substituted by hydroxy and/or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; and pharmaceutically acceptable salts thereof.

2. Compounds as claimed in claim 1, characterised by one of more of the following features:

a) $R_3$ and $R_5$ represent hydrogen;
b) n is one or two
c) R is located in positions 7 and/or 8; and
d) R is selected from halogeno, carboxy, loweralkoxycarbonyl, acylaminoloweralkyl, acyloxyloweralkyl and the carboxamido moiety as defined in claim 1.

3. Compounds as claimed in claim 1 or 2, wherein n is one, R is located in position 7 or 8 and is selected from loweralkoxycarbonyl, acyloxyloweralkyl and the carboxamido moiety as defined in claim 1.

4. Compounds as claimed in any one of claims 1 to 3, wherein, in the carboxamido moiety of the structure as defined in claim 1, A is straight or branched chain alkyl with up to 12 carbon atoms hydroxyloweralkyl, tetrazolyl or the grouping —E—$R_8$, wherein E is straight chain alkylene with up to three carbon atoms and is optionally substituted by hydroxy, and $R_8$ is phenyl, tetrazolyl or pyridyl; and B is hydrogen or loweralkyl.

5. Compounds as claimed in any one of claims 1 to 4, wherein, in the carboxamido moiety of the structure as defined in claim 1, A is tetrazolyl or the grouping —E—$R_8$, wherein E is methylene or ethylene and $R_8$ is phenyl or pyridyl; and B is hydrogen; the carboxamido moiety being in position 7.

6. A compound of any one of claims 1 to 5, namely 7 - (5 - tetrazolylaminocarbonyl) - 2,4 - (1*H*,3*H*,5*H*) - (1) - benzopyrano - (2,3 - d) - pyrimidinedione or 7 - (2 - [2 - pyridyl]ethylaminocarbonyl) - 2,4 - (1*H*,3*H*,5*H*) - (1) - benzopyrano - (2,3 - d) - pyrimidinedione.

7. Pharmaceutical compositions containing as active ingredient at least one compound as claimed in any one of claims 1 to 6 together with a pharmaceutical carrier or excipient.

8. Process for the preparation of a compound of the formula (I) as defined in claim 1, or of a pharmaceutically acceptable salt thereof; characterised in that one of steps A) to E) is applied:

A) for the preparation of compounds of formula I, wherein $R_5$ is hydrogen; reducing a compound of the formula

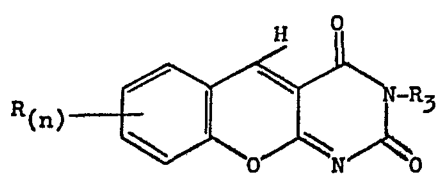

(IV)

wherein R, n and $R_3$ are as defined in Claim 1, with the proviso that when R is an electron-withdrawing radical then $R_3$ is hydrogen; or

B) for the preparation of compounds of formula I, wherein $R_3$ is hydrogen and R and $R_5$ are delimited as specified in the provisos hereinbelow; cyclising a compound of the formula

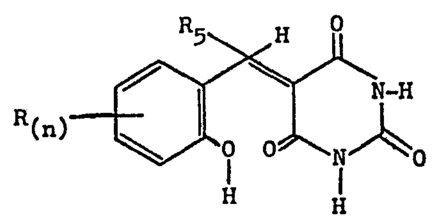

(VII)

wherein R is as defined in claim 1 with the proviso that it is not acyloxyloweralkyl or alkanoyloxy; and n and $R_5$ are as defined in claim 1 with the proviso that $R_5$ must be hydrogen when R is carboxy, acyl, formyl, acyl, formyl or haloloweralkyl; and wherein the hydroxy group in position 2 of the phenyl moiety may be protected; or

C) for the preparation of compounds of formula I, wherein R and n are as defined in claim 1 and $R_3$ and $R_5$ are both hydrogen, reacting a compound of the formula

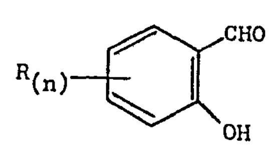

(II)

wherein R and n are as defined in claim 1, with barbituric acid in an alcoholic solvent and in the presence of a sulfonic acid; or

D) for the preparation of compounds of formula I, wherein R and n are as defined in claim 1, with the same proviso for substituent R as in process step B), and $R_3$ and $R_5$ are both hydrogen; reacting a compound of the formula

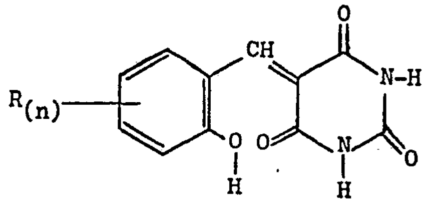

(VI)

in an alcoholic solvent and in the presence of a sulfonic acid;

E) for the preparation of compounds of formula I, wherein $R_5$ is loweralkyl; reacting a compound of the formula

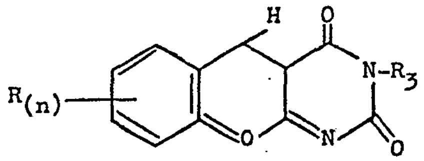

(IV)

with $R_5'MgHal$ in the present of a cuprous salt, wherein R, n and $R_3$ are as defined in claim 1, $R_5'$ is loweralkyl, and Hal is chlorine, bromine or iodine;

any of these steps A) to E) being followed by one or more of facultative finishing steps (i) to (xv) to convert a compound of formula I into another compound of formula I:

(i) esterification of the carboxyl group representing substituent R;
(ii) de-esterification of a loweralkoxycarbonyl group representing substituent R;
(iii) trans-esterification of a loweralkoxycarbonyl group representing substituent R;
(iv) acylation of any hydroxy group in/or representing substituent R;
(v) de-acylation of acyloxyloweralkyl, alkanoyloxy or acylaminoloweralkyl, representing substituent R;
(vi) reduction of an acyl group representing substituent R;
(vii) etherification of any hydroxyl group in/or representing substituent R;
(viii) hydrolysing, alkoholysing or reducing the cyano group representing substituent R;
(ix) oxydation of a hydroxymethyl group representing substituent R;
(x) acylation of an amino group in substituent R and, if desired, in situ or subsequently reducing the amide so obtained;
(xi) replacement of hydroxy by halogen in hydroxyloweralkyl representing substituent R;
(xii) amidation of the carboxyl group representing substituent R to obtain the carboxamido moiety as defined above;
(xiii) trans-amidation of a carboxamido moiety representing substituent R;
(xiv) de-amidation of a carboxamido moiety representing substituent R;
(xv) preparing a pharmaceutically acceptable salt of any of the compounds so obtained.

9. Process for the preparation of pharmaceutical compositions characterised in that a compound of formula I as defined in claim 1 or a pharmaceutically acceptable salt thereof is admixed with a pharmaceutical carrier or excipient.

**Revendications pour les Etats contractants: BE, CH, DE, FR, GB, IT, NL, SE**

1. Composés de la formule

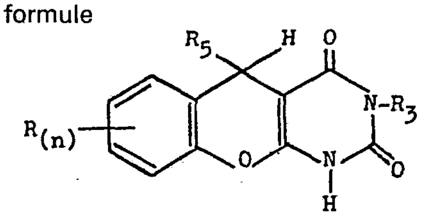

(I)

dans laquelle chacun de $R_3$ et $R_5$ représente de l'hydrogène ou un alcoyle inférieur, n est un nombre entier allant de 1 à 4 et R représente de l'hydrogène, un alcoyle inférieur, cycloalcoyle inférieur, cycloalcoyle inférieur alcoyle inférieur, acyloxyalcoyle inférieur, hydroxyalcoyle inférieur, alcoxy inférieur alcoyle inférieur, nitro, halogéno, haloalcoyle inférieur, hydroxy, alcoxy inférieur, acylaminoalcoyle inférieur, aminoalcoyle inférieur, mono- ou di-alcoyle inférieur aminoalcoyle inférieur, alcanoyloxy, carboxy, alcoxy inférieur carbonyle, acyle, formyle, cyano ou une partie carboxamido de la structure:

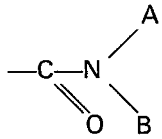

dans laquelle A est un groupe alcoyle à chaîne droite ou ramifiée ayant jusqu'à 12 atomes de carbone, un cycloalcoyle inférieur, cycloalcoyle inférieur alcoyle inférieur, alcoxy inférieur alcoyle inférieur, hydroxyalcoyle inférieur, fluoralcoyle inférieur, alcényle inférieur, alcoyle inférieur thioalcoyle inférieur, alcoyle inférieur sulfoxyalcoyle inférieur, alcoyle inférieur sulfonylalcoyle inférieur, thiazolyle, oxazolyle, thiadiazolyle, méthylthiadiazolyle, furyle, pyrazolyle, tétrazolyle, méthyltétrazolyle, hydroxy-pyrimidinyle, phényle, pyrimidinyl-dione, ou le groupement

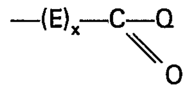

dans laquelle E est un alcoylène inférieur à chaîne droite ou ramifiée ou un alcoylène inférieur cyclique, x est égal à zéro ou 1 et Q représente un hydroxy, un alcoxy inférieur, un amino ou mono- ou dialcoyle inférieur amino; ou le groupement —E—$R_8$, dans lequel E est tel que précédemment défini et est éventuellement substitué par un hydroxy et/ou un phényle, $R_8$ représente un phényle, thiazolyle, oxazolyle, thiadiazolyle, méthylthiadiazolyle, tétrazolyle, méthyltétrazolyle, furyle, pyridyle, méthylpyridyle ou pipéridinyle; et B représente l'hydrogène, un alcoyle inférieur, un cycloalcoyle inférieur, un cycloalcoyle inférieur alcoyle inférieur, ou un alcényle inférieur; ou A et B, lorsqu'ils sont pris ensemble avec l'atome d'azote auquel ils sont rattachés, représentent un imidazolyle, morpholinyle, pyrrolidinyle, pipéridinyle ou pipérazinyle, lesdits cycles héterocycliques étant éventuellement substitués par un groupe hydroxy, alcoyle inférieur ou hydroxyalcoyle inférieur; et leurs sels pharmaceutiquement acceptables.

2. Composés selon la revendication 1, caractérisés par l'une ou plusieurs des caractéristiques suivantes:

a) $R_3$ et $R_5$ représentent l'hydrogène;
b) n est égal à 1 ou 2;
c) R est disposé aux positions 7 et/ou 8; et
d) R est choisi parmi un halogéno, carboxy, alcoxy inférieur carbonyle, acylaminoalcoyle inférieur, acyloxyalcoyle inférieur et la partie carboxamido telle que définie à la revendication 1.

3. Composés selon la revendication 1 ou 2, caractérisés en ce que n est égal à 1, R est disposé à la position 7 ou 8 et est choisi parmi un alcoxy inférieur carbonyle, acyloxyalcoyle inférieur et la partie carboxamido telle que définie à la revendication 1.

4. Composés selon l'une quelconque des revendications 1 à 3, caractérisés en ce que, dans la partie carboxamido de la structure telle que définie à la revendication 1, A est un groupe alcoyle à chaîne droite ou ramifiée ayant jusqu'à 12 atomes de carbone, une hydroxyalcoyle inférieur, tétrazolyle ou le groupement —E—$R_8$, dans lequel E est un groupe alcoylène à chaîne droite ayant jusqu'à 3 atomes de carbone et est éventuellement substitué par un hydroxy, et $R_8$ est un phényle, tétrazolyle ou pyridyle; et B est de l'hydrogène ou un groupe alcoyle inférieur.

5. Composés selon l'une quelconque des revendications 1 à 4, caractérisés en ce que, dans la partie carboxamido de la structure telle que définie à la revendication 1, A est un tétrazolyle ou bien le groupement —E—$R_8$, dans lequel E est un méthylène ou l'éthylène et $R_8$ est un phényle ou pyridyle; et B est de l'hydrogène; la partie carboxamido étant en position 7.

6. Composé selon l'une quelconque des revendications 1 à 5, caractérisé en ce qu'il est précisément la 7 - (5 - tétrazolylaminocarbonyl) - 2,4 - (1*H*,3*H*,5*H*) - (1) - benzopyrano - (2,3 - d) - pyrimidinedione ou la 7 - (2 - [2 - pyridyl]éthylaminocarbonyl) - 2,4 - (1*H*,3*H*,5*H*) - (1) - benzopyrano - (2,3 - d) - pyrimidinedione.

7. Compositions pharmaceutiques contenant comme ingrédient actif au moins un composé selon l'une quelconque des revendications 1 à 6 avec un support ou excipient pharmaceutique.

8. Procédé de préparation d'un composé de la formule (I) telle que définie à la revendication 1, ou un sel pharmaceutiquement acceptable de celui-ci; caractérisé en ce que l'on applique une des étapes A à E;

A) pour la préparation des composés de la formule I, dans laquelle $R_5$ est l'hydrogène; la réduction d'un composé de la formule

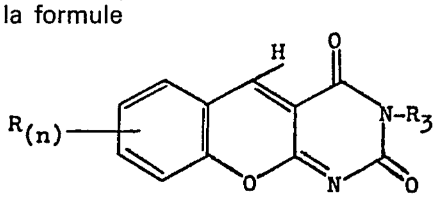

(IV)

dans laquelle R, n et $R_3$ sont tels que définis à la revendication 1; à la condition que lorsque R est un radical retirant un électron alors $R_3$ est l'hydrogène; ou

B) pour la préparation des composés de la formule I, dans laquelle $R_3$ est l'hydrogène et R et $R_5$ sont délimités comme spécifié dans les conditions ci-dessous; la cyclisation d'un composé de la formule:

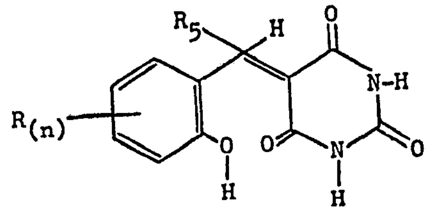

(VII)

dans laquelle R est tel que défini à la revendication 1 à la condition qu'il n'est pas un groupe acyloxyalcoyle inférieur ou alcanoyloxy; et n et $R_5$ sont tels que définis à la revendication 1 à la condition que $R_5$ doit être l'hydrogène lorsque R est un carboxy, acyle, formyle ou haloalcoyle inférieur; et dans laquelle le groupe hydroxy en position 2 de la partie phényle peut être protégé; ou

C) pour la préparation de composés de la formule I, dans laquelle R et n sont tels que définis à la revendication 1 et $R_3$ et $R_5$ sont à la fois l'hydrogène; la réaction d'un composé de la formule:

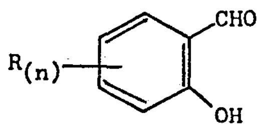

(II)

dans laquelle R et n sont tels que définis à la revendication 1, avec l'acide barbiturique dans un solvant alcoolique et en présence d'un acide sulfonique; ou

D) pour la préparation de composés de la formule I, dans laquelle R et n sont tels que définis à la revendication 1, avec la même condition pour le substituant R que dans l'étape B) de procédé, et $R_3$ et $R_5$ sont à la fois l'hydrogène; la réaction d'un composé de la formule:

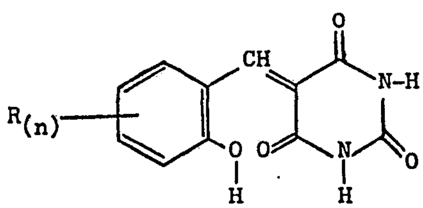

(VI)

dans un solvent alcoolique et en présence d'un acide sulfonique;

E) pour la préparation de composés de la formule I, dans laquelle $R_5$ est un alcoyle inférieur; la réaction d'un composé de la formule:

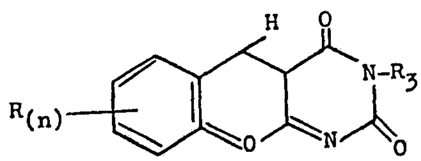

(IV)

avec $R_5'$MgHal en présence d'un sel cuivreux, dans laquelle R, n et $R_3$ sont tels que définis à la revendication 1, $R_5'$ est un alcoyle inférieur, et Hal est du chlore, du brome ou de l'iode;

n'importe laquelle de ces étapes A) à E) étant suivie par l'une ou plusieurs des étapes finales facultatives (i) à

(xv) pour convertir un composé de la formule I en un autre composé de la formule I:

(i) l'esterification du groupe carboxyle représentant le substituant R;

(ii) la dé-estérification d'un groupe alcoxy inférieur carbonyle représentant le substituant R;

(iii) la trans-estérification d'un groupe alcoxy inférieur carbonyl représentant le substituant R;

(iv) l'acylation de tout groupe hydroxy dans/ou représentant le substituant R;

(v) la dé-acylation du groupe acyloxyalcoyle inférieur, alcanoyloxy ou acylaminoalcoyle inférieur, représentant le substituant R;

(vi) la réduction d'un groupe acyle représentant le substituant R;

(vii) l'éthérification de tout groupe hydroxyle dans/ou représentant le substituant R;

(viii) l'hydrolysation, l'alcoolisation ou la réduction du groupe cyano représentant le substituant R;

(ix) l'oxydation d'un groupe hydroxyméthyle représentant le substituant R;

(x) l'acylation d'un groupe amino dans le substituant R et, si désiré, in situ ou subséquemment la réduction de l'amide ainsi obtenu;

(xi) le remplacement d'un hydroxy par un halogène dans un groupe hydroxyalcoyle inférieur représentant le substituant R;

(xii) l'amidation du groupe carboxyle représentant le substituant R pour obtenir la partie carboxamido telle que précédemment définie;

(xiii) la trans-amidation d'une partie carboxamido représentant le substituant R;

(xiv) la dé-amidation d'une partie carboxamido représentant le substituant R;

(xv) la préparation d'un sel pharmaceutique acceptable de n'importe lequel des composés ainsi obtenus.

9. Procédé pour la préparation de compositions pharmaceutiques caractérisé en ce qu'un composé de la formule I telle que définie à la revendication 1 ou un sel pharmaceutique acceptable de celui-ci est mélangé avec un support ou excipient pharmaceutique.

**Patentansprüche für die Vertragsstaaten: BE, CH, DE, FR, GB, IT, NL, SE**

1. Verbindungen der Formel

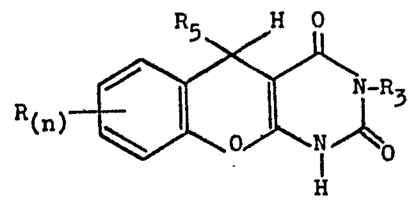

(I)

in der $R_3$ und $R_5$ jeweils für Wasserstoff oder niederes Alkyl stehen, n eine ganze Zahl von 1 bis 4 ist und R für Wasserstoff, Niederalkyl, Niedercycloalkyl, Niedercycloalkyl-niederalkyl, Acyloxy-niederalkyl, Hydroxy-niederalkyl, Niederalkoxy-niederalkyl, Nitro, Halogen, Halogen-niederalkyl, Hydroxy, Niederalkoxy, Acylamino-niederalkyl, Amino-niederalkyl, Mono- oder Di-niederalkylamino-niederalkyl, Alkanoyloxy, Carboxy, Niederalkoxycarbonyl, Acyl, Formyl, Cyan oder eine Carboxamidokomponente der Struktur

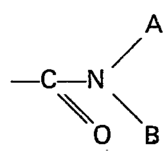

steht, in der A geradkettiges oder verzweigtes Alkyl mit bis zu 12 Kohlenstoffatomen, Niedercycloalkyl, Niedercycloalkyl-niederalkyl, Niederalkoxy-niederalkyl, Hydroxyniederalkyl, Fluorniederalkyl, Niederalkenyl, Niederalkylthio-niederalkyl, Niederalkylsulfoxy-niederalkyl, Niederalkylsulfonylniederalkyl, Thiazolyl, Oxazolyl, Thiadiazolyl, Methylthiadiazolyl, Furyl, Pyrazolyl, Tetrazolyl, Methyltetrazolyl, Hydroxypyrimidinyl, Phenyl, Pyrimidinyl-dion oder die Gruppierung

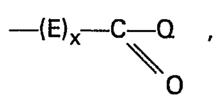

in der E geradkettiges oder verzweigtes niederes Alkylen oder cyclisches Niederalkylen, X null oder 1 und Q Hydroxy, Niederalkoxy, Amino oder Mono- oder Diniederalkylamino ist, oder die Gruppierung —E—$R_8$ ist, worin E die vorstehend genannte Bedeutung hat und wahlweise mit Hydroxy und/oder Phenyl substituiert ist, $R_8$ für Phenyl, Thiazolyl, Oxazolyl, Thiadiazolyl, Methylthiadiazolyl, Tetrazolyl, Methyltetrazolyl, Furyl, Pyridyl, Methylpyridyl oder Piperidinyl und B für Wasserstoff, Niederalkyl, Niedercycloalkyl, Niedercycloalkyl-niederalkyl oder Niederalkenyl steht oder A und B gemeinsam mit dem Stickstoffatom, an das sie gebunden sind, Imidazolyl, Morpholinyl, Pyrrolidinyl, Piperidinyl oder Piperazinyl darstellen, wobei diese heterocyclischen Ringe gegebenenfalls mit Hydroxy, Niederalkyl oder Hydroxyniederalkyl substituiert sind, und pharmazeutisch unbedenkliche Salze dieser Verbindungen.

2. Verbindungen nach Anspruch 1, gekennzeichnet, durch eines oder mehrere der folgenden Merkmale:

a) $R_3$ und $R_5$ stellen Wasserstoff dar;

b) n steht für 1 oder 2;

c) R steht in den Stellungen 7 und/oder 8 und

d) R ist aus Halogen, Carboxy, Niederalkyloxycarbonyl, Acylamino-niederalkyl, Acyloxy-niederalkyl und der in Anspruch 1 definierten Carboxamidokomponente ausgewählt.

3. Verbindungen nach Anspruch 1 oder 2, worin n für 1 steht und R in der Stellung 7 oder 8

steht und aus Niederalkoxycarbonyl, Acyloxyniederalkyl und der in Anspruch 1 definierten Carboxamidokomponente ausgewählt ist.

4. Verbindungen nach irgendeinem der Ansprüche 1 bis 3, dadurch gekennzeichnet, daß in der Carboxamidokomponente der in Anspruch 1 definierten Struktur A ein geradkettiger oder verzweigter Alkylrest mit bis zu 12 Kohlenstoffatome, Hydroxyniederalkyl, Tetrazolyl oder die Gruppierung —E—$R_8$ ist, worin E geradkettiges Alkylen mit bis zu drei Kohlenstoffatomen und wahlweise mit Hydroxy substituiert ist und $R_8$ Phenyl, Tetrazolyl oder Pyridyl ist und B für Wasserstoff oder Niederalkyl steht.

5. Verbindungen nach irgendeinem der Ansprüche 1 bis 4, dadurch gekennzeichnet, daß in der Carboxamidokomponente der in Anspruch 1 definierten Struktur A Tetraazolyl oder die Gruppierung —E—$R_8$ ist, worin E Methylen oder Äthylen und $R_8$ Phenyl oder Pyridyl ist und B für Wasserstoff steht, wobei die Carboxamidokomponente in 7-Stellung steht.

6. Eine Verbindung nach irgendeinem der Ansprüche 1 bis 5, nämlich 7 - (5 - Tetrazolylaminocarbonyl) - 2,4 - (1H,3H,5H) - (1) - benzopyran - (2,3 - d) - pyrimidindion oder 7 - (2 - [2 - Pyridyl] - äthylaminocarbonyl) - 2,4 - (1H,3H,5H) - (1) - benzopyran - (2,3 - d) - pyrimidindion.

7. Arzneimittelzubereitungen, enthaltend als aktives Ingrediens wenigstens eine Verbindung, wie sie in irgend- einem der Ansprüche 1 bis 6 beansprucht wird, zusammen mit einem pharmazeutischen Träger oder Hilfsstoff.

8. Verfahren zur Herstellung einer Verbindung der in Anspruch 1 definierten Formel (I) oder eines pharmazeutisch unbedenklichen Salzes dieser Verbindung, dadurch gekennzeichnet, daß einer der Schritte (A) bis (E) angewandt wird:

A) für die Herstellung von Verbindungen der Formel I, in der $R_5$ Wasserstoff ist: Reduktion einer Verbindung der Formel

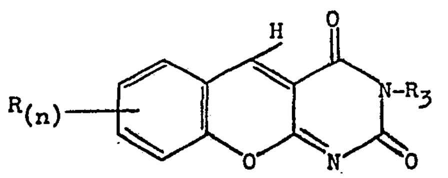

(IV)

in der R, n und $R_3$ die in Anspruch 1 genannten Bedeutungen haben, mit der Maßgabe, daß, wenn R ein elektronenanziehender Rest ist, $R_3$ Wasserstoff ist, oder

B) für die Herstellung von Verbindungen der Formel I, in der $R_3$ Wasserstoff ist und R und $R_5$ begrenzt sind, wie in den nachstehenden Maßgaben angegeben: Cyclisierung einer Verbindung der Formel

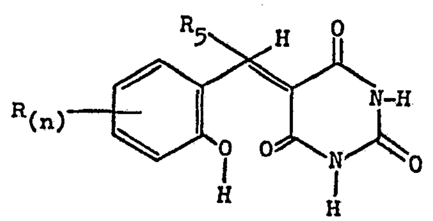

(VII)

in der R die in Anspruch 1 genannte Bedeutung hat, mit der Maßgabe, daß R nicht für Acyloxyniederalkyl oder Alkanoyloxy steht, und n und $R_5$ die in anspruch 1 genannten Bedeutungen haben mit der Maßgabe, daß $R_5$ Wasserstoff sein muß, wenn R Carboxy, Acyl, Formyl oder Halogen-niederalkyl ist, und worin die Hydroxylgruppe in 2-Stellung des Phenylrestes geschützt sein kann, oder

C) für die Herstellung von Verbindungen der Formel I, in der R und N die in Anspruch 1 definierten Bedeutungen haben und $R_3$ und $R_5$ beide Wasserstoff sind: Umsetzung einer Verbindung der Formel

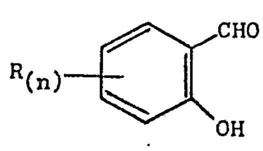

(II)

in der R und n die in Anspruch 1 definierten Bedeutungen haben, mit Barbitursäure in einem alkoholischen Lösungsmittel in Gegenwart einer Sulfonsäure oder

D) für die Herstellung von Verbindungen der Formel I, in der R und n die in Anspruch 1 definierten Bedeutungen haben mit dem gleichen Vorbehalt für den Substituenten R wie in der Verfahrensstufe B) und $R_3$ und $R_5$ beide Wasserstoff sind: Umsetzung einer Verbindung der Formel

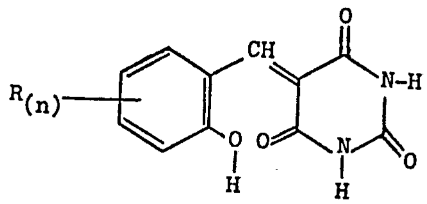

(VI)

in einem alkoholischen Lösungsmittel und in Gegenwart einer Sulfonsäure;

E) für die Herstellung von Verbindungen der Formel I, in der $R_5$ Niederalkyl ist: Umsetzung einer Verbindung der Formel

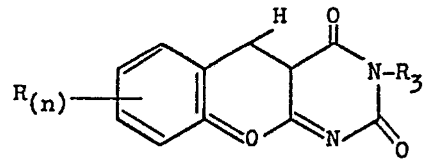

(IV)

mit $R_5'$MgHal in Gegenwart eines Kupfer(I)-salzes, wobei R, n und $R_3$ die in Anspruch 1 definierten Bedeutungen haben, $R_5'$ Niederalkyl und Hal Chlor, Brom oder lod ist,

und wobei auf alle diese Stufen (A) bis (E) eine oder mehrere wahlfreie Fertigstellungsstufen (I) bis (XV) folgen, um eine Verbindung der Formel I in eine andere Verbindung der Formel I umzuwandeln;

I) Veresterung der Carboxylgruppe, die den Substituenten R darstellt;

II) Entesterung einer Niederalkoxycarbonylgruppe, die den Substituenten R darstellt;

III) Umesterung eine Niederalkoxycarbonylgruippe, die den Substituenten RT darstellt,

IV) Acylierung einer beliebigen Hydroxylgruppe, die im Substituenten R vorhanden ist oder diesen darstellt;

V) Deacylierung von Acyloxy-niederalkyl, Alkanoyloxy oder Acylamino-niederalkyl, die den Substituenten R darstellen;

VI) Reduktion einer Acylgruppe, die den Substituenten R darstellt;

VII) Verätherung einer beliebigen Hydroxylgruppe, die im Substituenten R vorhanden ist oder diesen darstellt;

VIII Hydrolyse, Alkoholysierung oder Reduktion der den Substituenten R darstellenden Cyangruppe;

IX) Oxidation einer den Substituenten R darstellenden Hydroxymethylgruppe;

X) Acylierung einer Aminogruppe im Substituenten R und gegebenenfalls In-situ-Reduktion oder anschließende Reduktion des so erhaltenen Amids;

XI) Ersatz von Hydroxy durch Halogen im Hydroxy-niederalkyl, das den Substituenten R darstellt;

XII) Amidierung der den Substituenten R darstellenden Carboxylgruppe zur Bildung der vorstehend definierten Carboxamidokomponente;

XIII) Transamidierung einer den Substituenten R darstellenden Carboxamidokomponente;

XIV) Desamidierung einer den Substituenten R darstellenden Carboxamidokomponente;

XV) Herstellung eines pharmazeutisch unbedenklichen Salzes beliebiger Verbindungen, die in dieser Weise erhalten worden sind.

9. Verfahren zur Herstellung von Arzneimittelzubereitungen, dadurch gekennzeichnet, daß eine Verbindung der in Anspruch 1 definierten Formel (I) oder ein pharmazeutisch unbedenkliches Salz dieser Verbindung mit einem pharmazeutischen Träger oder Hilfsstoff gemischt wird.

**Claim for the Contracting State: AT**

1. Process for the preparation of a compound of the formula

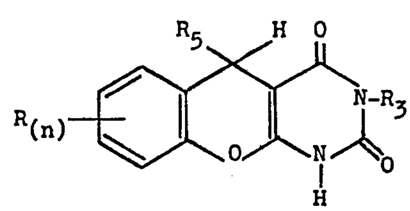

**(I)**

wherein each of $R_3$ and $R_5$ represents hydrogen or loweralkyl, n is an integer from one to four and R represents hydrogen, loweralkyl, lowercycloalkyl, lowercycloalkylloweralkyl, acyloxyloweralkyl, hydroxyloweralkyl, loweralkoxyloweralkyl, nitro, halogeno, haloloweralkyl, hydroxy, loweralkoxy, acylaminoloweralkyl, aminoloweralkyl, mono- or diloweralkylaminoloweralkyl, alkanoyloxy, carboxy, loweralkoxycarbonyl, acyl, formyl, cyano or a carboxamido moiety of the structure

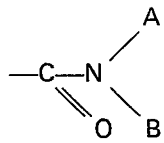

wherein A is straight or branched chain alkyl with up to 12 carbon atoms, lowercycloalkyl, lowercycloalkylloweralkyl, loweralkoxylloweralkyl, hydroxyloweralkyl, fluoroloweralkyl, loweralkenyl, loweralkylthioloweralkyl, loweralkylsulfoxyloweralkyl, loweralkylsulfonylloweralkyl, thiazolyl, oxazolyl, thiadiazolyl, methylthiadiazolyl, furyl, pyrazolyl, tetrazolyl, methyltetrazolyl, hydroxypyrimidinyl, phenyl, pyrimidinyl-dione, or the grouping

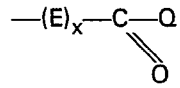

wherein E is a straight or branched chain loweralkylene or cyclic loweralkylene, X is zero or one and Q is hydroxy, loweralkoxy, amino or mono- or diloweralkylamino; or the grouping —E—$R_8$, wherein E is as defined above and is optionally substituted by hydroxy and/or phenyl, $R_8$ is phenyl, thiazolyl, oxazolyl, thiadiazolyl, methylthiadiazolyl, tetrazolyl, methyltetrazolyl, furyl, pyridyl, methylpyridyl or piperidinyl; and B is hydrogen, loweralkyl, lowercycloalkyl, lowercycloalkylloweralkyl, or loweralkenyl; or A and B, when taken together with the nitrogen atom to which they are attached, represent imidazolyl, morpholinyl, pyrrolidinyl, piperidinyl or piperazinyl said heterocyclic rings being optionally substituted by hydroxy, loweralkyl or hydroxyloweralkyl; or of a pharmaceutically acceptable salt thereof; characterised in that one of steps A to E is applied:

A) for the preparation of compounds of formula I, wherein $R_5$ is hydrogen; reducing a compound of the formula

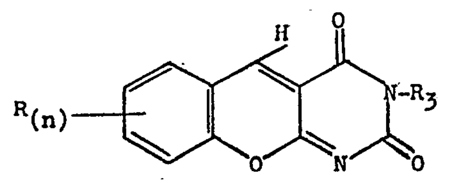

(IV)

wherein R, n and $R_3$ are as defined above; with the proviso that when R is an electron-withdrawing radical then $R_3$ is hydrogen; or

B) for the preparation of compounds of formula I, wherein $R_3$ is hydrogen and R and $R_5$ are delimited as specified in the provisos hereinbelow; cyclising a compound of the formula

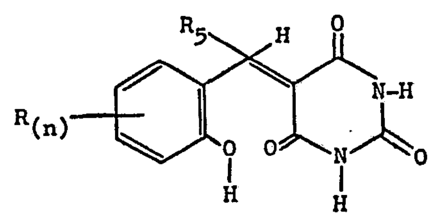

(VII)

wherein R is as defined above with the proviso that it is not acyloxyloweralkyl or alkanoyloxy; and n and $R_5$ are as defined above with the proviso that $R_5$ must be hydrogen when R is carboxy, acyl, formyl or haloloweralkyl; and wherein the hydroxy group in position 2 of the phenyl moiety may be protected; or

C) for the preparation of compounds of formula I, wherein R and n are as defined above and $R_3$ and $R_5$ are both hydrogen, reacting a compound of the formula

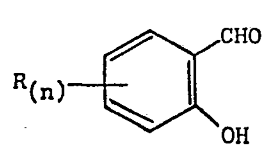

(II)

wherein R and n are as defined above, with barbituric acid in an alcoholic solvent and in the presence of a sulfonic acid; or

D) for the preparation of compounds of formula I, wherein R and n are as defined above, with the same proviso for substituent R as in process step B), and $R_3$ and $R_5$ are both hydrogen; reacting a compound of the formula

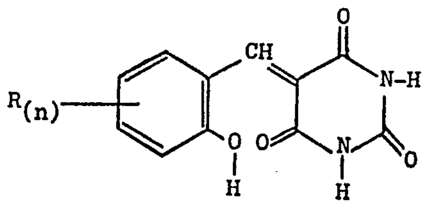

(VI)

in an alcoholic solvent and in the presence of a sulfonic acid;

E) foir the preparation of compounds of formula I, wherein $R_5$ is loweralkyl; reacting a compound of the formula

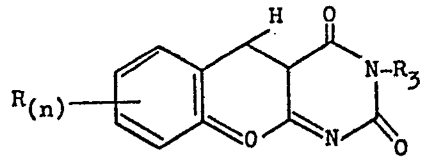

(IV)

with $R_5'MgHal$ in the presence of a cuprous salt, wherein R, n and $R_3$ are as defined above, $R_5'$ is loweralkyl, and Hal is chlorine, bromine or iodine;

any of these steps A) to E) being followed by one or more of facultative finishing steps (i) to (xv) to convert a compound of formula I into another compound of formula I:

(i) esterification of the carboxyl group representing substituent R;
(ii) de-esterification of a loweralkoxycarbonyl group representing substituent R;
(iii) trans-esterification of a loweralkoxycarbonyl group representing substituent R;
(iv) acylation of any hydroxy group in/or representing substituent R;
(v) de-acylation of acyloxyloweralkyl, alkanoyloxy or acylaminoloweralkyl, representing substituent R;
(vi) reduction of an acyl group representing substituent R;
(vii) etherification of any hydroxyl group in/or representing substituent R;
(viii) hydrolysing, alkoholysing or reducing the cyano group representing substituent R;
(ix) oxydation of a hydroxymethyl group representing substituent R;
(x) acylation of an amino group in substituent R and, if desired, in situ or subsequently reducing the amide so obtained;
(xi) replacement of hydroxy by halogen in hydroxyloweralkyl representing substituent R;
(xii) amidation of the carboxyl group representing substituent R to obtain the carboxamido moiety as defined above;
(xiii) trans-amidation of a carboxamido moiety representing substituent R;
(xiv) de-amidation of a carboxamido moiety representing substituent R;
(xv) preparing a pharmaceutically acceptable salt of any of the compounds so obtained.

2. Process as claimed in claim 1, characterised in that 7 - (5 - tetrazolylaminocarbonyl) - 2,4 - (1*H*,3*H*,5*H*) - (1) - benzopyrano - (2,3 - d) - pyrimidinedione or a pharmaceutically acceptable salt thereof is prepared.

3. Process as claimed in claim 1 or 2, characterised in that in step A) reduction is effected with sodium borohydride or sodium cyanoborohydride in an alcoholic medium.

4. Process as claimed in claim 1 or 2, characterised in that in step C) and D) the reaction is carried out in n-propanol, n-butanol or iso-propanol in the presence of methanesulfonic acid or p-toluenesulfonic acid.

5. Process for the preparation of pharmaceutical compositions characterised in that a compound of formula I as defined in claim 1 or a pharmaceutically acceptable salt thereof is admixed with a pharmaceutical carrier or excipient.

6. Process for the preparation of pharmaceutical compositions characterised in that a compound of formula I or a pharmaceutically acceptable salt thereof, whenever obtained by the process as claimed in any one of claims 1 to 4, is admixed with a pharmaceutical carrier or excipient.

**Revendications pour l'Etat contractant: AT**

1. Procédé de préparation d'un composé de la formule

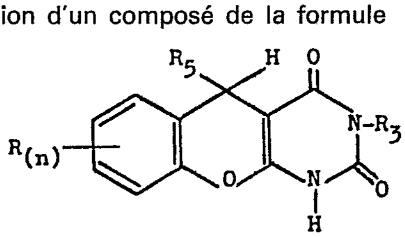

(I)

dans laquelle chacun de $R_3$ et $R_5$ représente de l'hydrogène ou un alcoyle inférieur, n est un nombre entier allant de 1 à 4 et R représente de l'hydrogène, un alcoyle inférieur, cycloalcoyle inférieur, cycloalcoyle inférieur alcoyle inférieur, acyloxyalcoyle inférieur, hydrosyalcoyle inférieur, alcoxy inférieur alcoyle inférieur, nitro, halogéno, haloalcoyle inférieur, hydroxy, alcoxy inférieur, acylaminoalcoyle inférieur, aminoalcoyle inférieur, mono- ou di-alcoyle inférieur aminoalcoyle inférieur, alcanoyloxy, carboxy, alcoxy inférieur carbonyle, acyle, formyle, cyano ou une partie carboxamido de la structure:

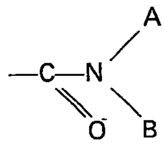

dans laquelle A est un groupe alcoyle à chaîne droite ou ramifiée ayant jusqu'à 12 atomes de carbone, un cycloalcoyle inférieur, cycloalcoyle inférieur alcoyle inférieur, alcoxy inférieur alcoyle inférieur, hydroxyalcoyle inférieur, fluoroalcoyle inférieur, alcényle inférieur, alcoyle inférieur thioalcoyle inférieur, alcoyle inférieur sulfoxyalcoyle inférieur, alcoyle inférieur sulfonylalcoyle inférieur, thiazolyle, oxazolyle, thiadiazolyle, méthylthiadiazolyle, furyle, pyrazolyle, tétrazolyle, méthyltétrazolyle, hydroxy-pyrimidinyle, phényle, pyrimidinyl-dione, ou le groupement

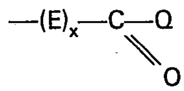

dans laquelle E est un alcoylène inférieur à chaîne droite ou ramifiée ou un alcoylène inférieur cyclique, x est égal à zéro ou 1 et Q représente un hydroxy, un alcoxy inférieur, un amino ou mono- ou dialcoyle inférieur amino; ou le groupement —E—$R_8$, dans lequel E est tel que précédemment défini et est éventuellement substitué par un hydroxy et/ou un phényle, $R_8$ représente un phényle, thiazolyle, oxazolyle, thiadiazolyle, méthylthiadiazolyle, tétrazolyle, méthyltétrazolyle, furyle, pyridyle, méthylpyridyle ou pipéridinyle; et B représente l'hydrogène, un alcoyle inférieur, un cycloalcoyle inférieur, un cycloalcoyle inférieur alcoyle inférieur, ou un alcényle inférieur; ou A et B, lorsqu'ils sont pris ensemble avec l'atome d'azote auquel ils sont rattachés, représentent un imidazolyle, morpholinyle, pyrrolidinyl, pipéridinyle ou pipérazinyle, lesdits cycles, hétérocycliques étant éventuellement substitués par un groupe hydroxy, alcoyle inférieur ou hydroxyalcoyle inférieur; ou un sel pharmaceutiquement acceptable de celui-ci; caractérisé en ce que l'on applique l'une des étapes A à E:

A) pour la préparation des composés de la formule I, dans laquelle $R_5$ est l'hydrogène; la réduction d'un composé de la formule:

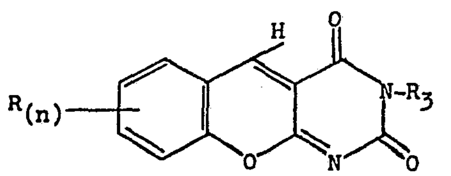

(IV)

dans laquelle R, n et $R_3$ sont tels que définis ci-dessus; à la condition que lorsque R est un radical retirant un électron alors $R_3$ est l'hydrogène; ou

B) pour la préparation des composés de la formule I, dans laquelle $R_3$ est l'hydrogène et R et $R_5$ sont délimités comme spécifié dans les conditions ci-dessus; la cyclisation d'un composé de la formule:

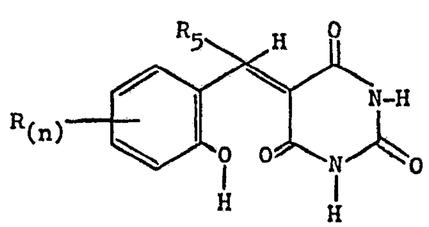

(VII)

dans laquelle R est tel que défini ci-dessus à la condition qu'il n'est pas un groupe acyloxyalcoyle inférieur ou alcanoyloxy; et n et $R_5$ sont tels que définis ci-dessus à la condition que $R_5$ doit être l'hydrogène lorsque R est un carboxy, acyle, formyle ou haloalcoyle inférieur; et dans laquelle le groupe hydroxy en position 2 de la partie phényle peut être protégé; ou

C) pour la préparation de composés de la formule I, dans laquelle R et n sont tels que définis ci-dessus et $R_3$ et $R_5$ sont à la fois l'hydrogène, la réaction d'un composé de la formule:

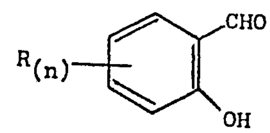

(II)

dans laquelle R et n sont tels que définis ci-dessus, avec l'acide barbiturique dans un solvant alcoolique et en présence d'un acide sulfonique; ou

D) pour la préparation de composés de la formule I, dans laquelle R et n sont tels que définis ci-dessus, avec la même condition pour le substituant R que dans l'etape B) de procédé, et $R_3$ et $R_5$ sont à la fois l'hydrogène; la réaction d'un composé de la formule:

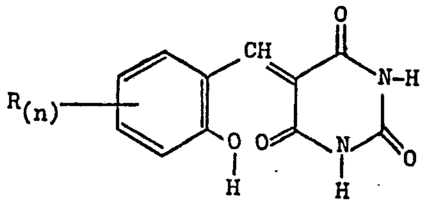

(VI)

dans un solvant alcoolique et en présence d'un acide sulfonique;

E) pour la préparation des composés de la formule I, dans laquelle $R_5$ est un alcoyle inférieur; la réaction d'un composé de la formule:

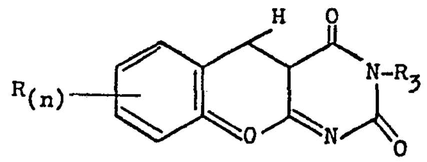

(IV)

avec $R_5'$MgHal en présence d'un sel cuivreux, dans laquelle R, n et $R_3$ sont tels que définis ci-dessus, $R_5'$ est un alcoyle inférieur, et Hal est du chlore, du brome ou de l'iode;

n'importe laquelle de ces étapes A) à E) étant suivie par l'une ou plusieurs des étapes finales facultatives (i) à (xv) pour convertir un composé de la formule I en un autre composé de la formula I:

(i) l'estérification du groupe carboxyle représentant le substituant R;

(ii) la dé-estérification d'un groupe alcoxy inférieur carbonyle représentant le substituent R;

(iii) la trans-estérification d'un groupe alcoxy inférieur carbonyl représentant le substituant R;

(iv) l'acylation de tout groupe hydroxy dans/ou représentant le substituant R;

(v) la dé-acylation du groupe acyloxyalcoyle inférieur, alcanoyloxy ou acylaminoalcoyle inférieur, représentant le substituant R;

(vi) la réduction d'un groupe acyle représentant le substituant R;

(vii) l'éthérification de tout groupe hydroxyle dans/ou représentant le substituant R;

(viii) l'hydrolysation, l'alcoolisation ou la réduction du groupe cyano représentant le substituant R;

(ix) l'oxydation d'un groupe hydroxyméthyle représentant le substituant R;

(x) l'acylation d'un groupe amino dans le substituant R et, si désiré, in situ ou subséquemment la réduction de l'amide ainsi obtenu;

(xi) le remplacement d'un hydroxy par un halogène dans un groupe hydroxyalcoyl inférieur représentant le substituant R;

(xii) l'amidation du groupe carboxyle représentant le substituant R pour obtenir la partie carboxamido telle que précédemment définie;

(xiii) la trans-amidation d'une partie carboxamido représentant le substituant R;

(xiv) la dé-amidation d'une partie carboxamido représentant le substituant R;

(xv) la préparation d'un sel pharmaceutique acceptable de n'importe lequel des composés ainsi obtenus.

2. Procédé selon la revendication 1, caractérisé en ce que l'on prépare la 7 - (5 - tétrazolyl-aminocarboxyl)2,4 - (1*H*,3*H*,5*H*) - (1) - benzopyrano - (2,3 - d) - pyrimidinedione ou un sel pharmaceutiquement acceptable de celle-ci.

3. Procédé selon la revendication 1 ou 2, caractérisé en ce que dans l'étape A) on effectue la réduction avec du borohydrure de sodium ou du cyanoborohydrure de sodium dans un milieu alcoolique.

4. Procédé selon la revendication 1 ou 2, caractérisé en ce que dans l'étape C) et D) on réalise la réaction dans du n-propanol, n-butanol ou isopropanol en présence d'un acid méthanesulfonique ou d'acide p-toluènesulfonique.

5. Procédé pour la préparation de compositions pharmaceutiques, caractérisé en ce qu'on mélange un composé de la formule I telle que définie à la revendication 1 ou un sel pharmaceutiquement acceptable de celui-ci avec un support ou excipient pharmaceutique.

6. Procédé pour la préparation de compositions pharmaceutiques, caractérisé en ce qu'on

mélange un compose de la formule I ou un sel pharmaceutiquement acceptable de celui-ci, pour autant qu'il soit obtenu par la procéde selon l'une quelconque des revendications 1 à 4, avec un support ou excipient pharmaceutique.

**Patentansprüche für den Vertragsstaat: AT**

1. Verfahren zur Herstellung einer Verbindung der Formel

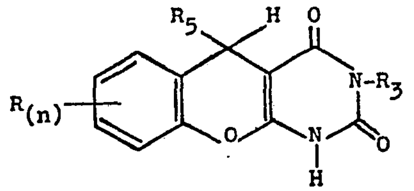

(I)

in der $R_3$ und $R_5$ jeweils für Wasserstoff oder niederes Alkyl stehen, n eine ganze Zahl von 1 bis 4 ist und R für Wasserstoff, Niederalkyl, Niedercycloalkyl, Niedercycloalkyl-niederalkyl, Acyloxy-niederalkyl, Hydroxy-niederalkyl, Niederalkoxy-niederalkyl, Nitro, Halogen, Halogen-niederalkyl, Hydroxy, Niederalkoxy, Acylamino-niederalkyl, Amino-niederalkyl, Mono- oder Di-niederalkylamino-niederalkyl, Alkanoyloxy, Carboxy, Niederalkoxycarbonyl, Acyl, Formyl, Cyan oder eine Carboxamidokomponente der Struktur

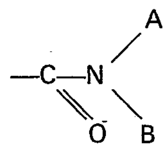

steht, in der A geradkettiges oder verzweigtes Alkyl mit bis zu 12 Kohlenstoffatomen, Niedercycloalkyl, Niedercycloalkyl-niederalkyl, Niederalkoxy-niederalkyl, Hydroxyniederalkyl, Fluorniederalkyl, Niederalkenyl, Niederalkylthio-niederalkyl, Niederalkylsulfoxy-niederalkyl, Niederalkylsulfonylniederalkyl, Thiazolyl, Oxazolyl, Thiadiazolyl, Methylthiadiazolyl, Furyl, Pyrazolyl, Tetrazolyl, Methyltetrazolyl, Hydroxypyrimidinyl, Phenyl, Pyrimidinyl-dion oder die Gruppierung

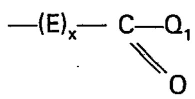

in der E geradkettiges oder verzweigtes niederes Alkylen oder cyclisches Niederalkylen, X null oder 1 und Q Hydroxy, Niederalkoxy, Amino oder Mono- oder Diniederalkylamino ist, oder die Gruppierung $-E-R_8$ ist, worin E die vorstehend genannte Bedeutung hat und wahlweise mit Hydroxy und/oder Phenyl substituiert ist, $R_8$ für Phenyl, Thiazolyl, Oxazolyl, Thiadiazolyl, Methylthiadiazolyl, Tetrazolyl, Methyltetrazolyl, Furyl, Pyridyl, Methylpyridyl oder Piperidinyl und B für Wasserstoff, Niederalkyl, Niedercycloalkyl, Niedercycloalkyl-niederalkyl oder Niederalkenyl steht oder A und B gemeinsam mit dem Stickstoffatom, an das sie gebunden sind, Imidazolyl, Morpholinyl, Pyrrolidinyl, Piperidinyl oder Piperazinyl darstellen, wobei diese heterocyclischen Ringe gegebenenfalls mit Hydroxy, Niederalkyl oder Hydroxyniederalkyl substituiert sind, und eines pharmazeutisch unbedenklichen Salzes dieser Verbindungen, dadurch gekennzeichnet, daß man eine der folgenden Stufen A bis E anwendet:

A) für die Herstellung von Verbindungen der Formel I, in der $R_5$ Wasserstoff ist: Reduktion einer Verbindung der Formel

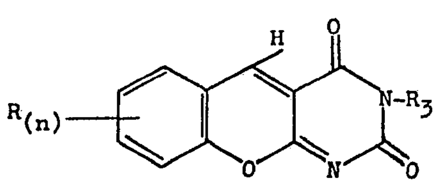

(IV)

in der R, n und $R_3$ die vorstehend genannten Bedeutungen haben, mit der Maßgabe, daß, wenn R ein elektronenanziehender Rest ist, $R_3$ Wasserstoff ist, oder

B) für die Herstellung von Verbindungen der Formel I, in der $R_3$ Wasserstoff ist und R und $R_5$ begrenzt sind, wie in den nachstehenden Maßgaben angegeben: Cyclisierung einer Verbindung der Formel

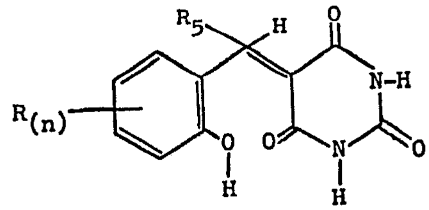

(VII)

in der R die vorstehend genannte Bedeutung hat, mit der Maßgabe, daß R nicht für Acyloxyniederalkyl oder Alkanoyloxy steht, und n und $R_5$ die vorstehend genannten Bedeutungen haben mit der Maßgabe, daß $R_5$ Wasserstoff sein muß, wenn R Carboxy, Acyl, Formyl oder Halogenniederalkyl ist, und worin die Hydroxylgruppe in 2-Stellung des Phenylrestes geschützt sein kann, oder

C) für die Herstellung von Verbindungen der Formel I, in der R und n die vorstehend genannten Bedeutungen haben und $R_3$ und $R_5$ beide Wasserstoff sind: Umsetzung einer Verbindung der Formel

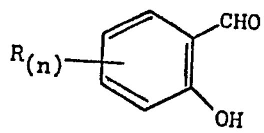

(II)

in der R und n die vorstehend genannten Bedeutungen haben, mit Barbitursäure in einem alkoholischen Lösungsmittel in Gegenwart einer Sulfonsäure oder

D) für die Herstellung von Verbindungen der Formel I, in der R und n die vorstehend genannten Bedeutungen haben mit dem gleichen Vorbehalt für den Substituenten R wie in der Verfahrensstufe B) und $R_3$ und $R_5$ beide Wasserstoff sind: Umsetzung einer Verbindung der Formel

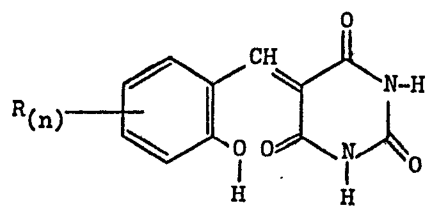

(VI)

in einem alkoholischen Lösungsmittel und in Gegenwart einer Sulfonsäure;

E) für die Herstellung von Verbindungen der Formel I, in der $R_5$ Niederalkyl ist: Umsetzung einer Verbindung der Formel

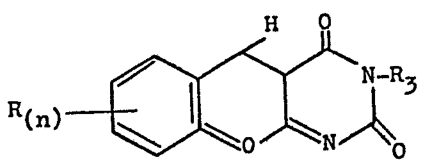

(IV)

mit $R_5'$MgHal in Gegenwart eines Kupfer(I)-salzes, wobei R, n und $R_3$ die vorstehend genannten Bedeutungen haben, $R_5'$ Niederalkyl und Hal Chlor, Brom oder Iod ist,

und wobei auf alle diese Stufen (A) bis (E) eine oder mehrere wahlfreie Fertigstellungsstufen (I) bis (XV) folgen, um eine Verbindung der Formel I in eine andere Verbindung der Formel I umzuwandeln:

I) Veresterung der Carboxylgruppe, die den Substituenten R darstellt;

II) Entesterung einer Niederalkoxycarbonylgruppe, die den Substituenten R darstellt;

III) Umesterung einer Niederalkoxycarbonylgruppe, die den Substituenten R darstellt;

IV) Acylierung einer beliebigen Hydroxylgruppe, die im Substituenten R vorhanden ist oder diesen darstellt;

V) Deacylierung von Acyloxy-niederalkyl, Alkanoyloxy oder Acylamino-niederalkyl, die den Substituenten R darstellen;

VI) Reduktion einer Acylgruppe, die den Substituenten R darstellt;

VII) Verätherung einer beliebigen Hydroxylgruppe, die im Substituenten R vorhanden ist oder diesen darstellt;

VIII) Hydrolyse, Alkoholysierung oder Reduktion der den Substituenten R darstellenden Cyangruppe;

IX) Oxidation einer den Substituenten R darstellenden Hydroxymethylgruppe;

X) Acylierung einer Aminogruppe im Substituenten R und gegebenenfalls In-situ-Reduktion oder anschließende Reduktion des so erhaltenen Amids;

XI) Ersatz von Hydroxy durch Halogen im Hydroxy-niederalkyl, das den Substituenten R darstellt;

XII) Amidierung der den Substituenten R darstellenden Carboxylgruppe zur Bildung der vorstehend definierten Carboxamidokomponente;

XIII) Transamidierung einer den Substituenten R darstellenden Carboxamidokomponente;

XIV) Desamidierung einer den Substituenten R darstellenden Carboxamidokomponente;
XV) Herstellung eines pharmazeutisch unbedenklichen Salzes beliebiger Verbindungen, die in dieser Weise erhalten worden sind.

2. Verfahren nach Anspruch 1, dadurch gekennzeichnet, daß 7 - (5 - Tetrazolylaminocarbonyl) - 2,4 - (1H,3H,5H) - (1) benzopyran - (2,3 - d) - pyrimidindion oder ein pharmazeutisch unbedenkliches Salz dieser Verbindung hergestellt wird.

3. Verfahren nach Anspruch 1 oder 2, dadurch gekennzeichnet, daß in der Stufe (A) die Reduktion mit Natriumborhydrid oder Natriumcyanborhydrid in einem alkoholischen Medium durchgeführt wird.

4. Verfahren nach Anspruch 1 oder 2, dadurch gekennzeichnet, daß in der Stufe (C) und (D) die Reaktion in n-Propanol, n-Butanol oder Isopropanol in Gegenwart von Methansulfonsäure oder p-Toluolsulfonsäure durchgeführt wird.

5. Verfahren zur Herstellung von Arzneimittelzubereitungen, dadurch gekennzeichnet, daß eine Verbindung der in Anspruch 1 definierten Formel I oder ein pharmazeutisch unbedenkliches Salz dieser Verbindung mit einem pharmazeutischen Träger oder Hilfsstoff gemischt wird.

6. Verfahren zur Herstellung von Arzneimittelzubereitungen, dadurch gekennzeichnet, daß eine Verbindung der Formel I oder ein pharmazeutisch unbedenkliches Salz dieser Verbindung, die nach dem in irgendeinem der Ansprüche 1 bis 4 beanspruchten Verfahren erhalten worden sind, mit einem pharmazeutischen Träger oder Hilfsstoff gemischt wird.